(19) Europäisches Patentamt
European Patent Office
Office européen des brevets

(11) **EP 3 285 721 B1**

(12) **EUROPEAN PATENT SPECIFICATION**

(45) Date of publication and mention of the grant of the patent:
**31.08.2022 Bulletin 2022/35**

(21) Application number: **16720281.1**

(22) Date of filing: **22.04.2016**

(51) International Patent Classification (IPC):
***A61K 8/31*** *(2006.01)* ***A61K 8/35*** *(2006.01)*
***A61K 8/37*** *(2006.01)* ***A61K 8/46*** *(2006.01)*
***A61Q 5/02*** *(2006.01)* ***A61K 8/04*** *(2006.01)*

(52) Cooperative Patent Classification (CPC):
**A61K 8/046; A61K 8/31; A61K 8/35; A61K 8/37; A61K 8/463; A61Q 5/02**

(86) International application number:
**PCT/US2016/028730**

(87) International publication number:
**WO 2016/172402 (27.10.2016 Gazette 2016/43)**

(54) **LOW VISCOSITY HAIR CARE COMPOSITION**

HAARPFLEGEMITTEL MIT NIEDRIGER VISKOSITÄT

COMPOSITION DE SOIN CAPILLAIRE À FAIBLE VISCOSITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2015 US 201562151641 P**

(43) Date of publication of application:
**28.02.2018 Bulletin 2018/09**

(73) Proprietor: **The Procter & Gamble Company**
**Cincinnati, OH 45202 (US)**

(72) Inventors:
- **THOMPSON, Todd, Ryan**
  **Cincinnati, Ohio 45202 (US)**
- **KOENIG, Peter, Herbert**
  **Cincinnati, Ohio 45202 (US)**
- **GLENN, Robert, Wayne, Jr.**
  **Cincinnati, Ohio 45202 (US)**
- **EIKE, David, Michael**
  **Cincinnati, Ohio 45202 (US)**
- **HUTTON, Howard, David, III**
  **Cincinnati, Ohio 45202 (US)**

(74) Representative: **P&G Patent Germany**
**Procter & Gamble Service GmbH**
**Sulzbacher Straße 40**
**65824 Schwalbach am Taunus (DE)**

(56) References cited:
**CA-A1- 2 078 375**
**JP-B2- 3 069 802**
**US-A1- 2005 201 967**
**US-A1- 2006 057 075**
**US-A1- 2011 319 790**
**US-A1- 2014 039 066**
**US-A1- 2015 071 977**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 285 721 B1

## Description

### FIELD OF THE INVENTION

Described herein is a hair care composition comprising a viscosity reducing agent having a partition dispersion coefficient of from about 0.05 to about 2.0, and a method of using the same.

### BACKGROUND OF THE INVENTION

Compact cleansing compositions comprising high surfactant content have certain advantages compared to traditional product forms. Firstly, compact cleansing compositions are more sustainable (lower carbon footprint and less waste) because they require less packaging per cleansing dose, less transportation, and lower storing costs. Aside from the sustainability benefit, such concentrated cleansing compositions enable non-traditional delivery forms to consumers such as the delivery of shampoos in a foam form (via aerosol or non-aerosol forms), which represents an attractive consumer form.

Given the low density of the foam, high concentration of surfactant is required to deliver sufficient amount of detersive surfactant for each use. However, high surfactant liquid cleansing compositions often exhibit high viscosity, which makes it prohibitive to deliver with a typical pump foam dispenser or a typical aerosol foam dispenser. Based on the foregoing, there is a need for a low viscosity concentrated liquid cleansing composition for delivery as foam.

In addition, compact cleansing compositions can also comprise surfactant soluble benefits agents such as perfumes, coloring agents, humectants, scalp and hair moisturizers, anti-frizz agents, anti-static agents, conditioning agents, anti-dandruff agents, UV filters, scalp barrier materials, styling agents, and other benefit agents that provide various benefits. Surfactant-soluble benefit agents have the tendency to remain more in the aqueous phase during hair cleansing and rinsing, deposit relatively less on hair surfaces, and end up in the waste water. Based on the foregoing, there is a need for improved deposition of surfactant-soluble benefit agents from concentrated shampoos. US patent application US 2015/0071977 A1 discloses shampoo compositions with high amount of sulfate surfactants comprising fragrance oils and the viscosity reducing agent isopropyl myristate.

### SUMMARY OF THE INVENTION

Described herein is a hair care composition comprising (a) from 0.2% to 10% of one or more surfactant soluble active agents, by weight of the hair care composition, wherein the surfactant soluble active agent is a perfume oil, wherein the total amount of one or more surfactant soluble active agents is from 0.2% to 10%, by weight of the hair care composition; (b) from 4% to 7% of one or more viscosity reducing agents, by weight of the hair care composition, having a partition dispersion coefficient of from 0.05 to 2.0, wherein the one or more viscosity reducing agents comprises limonene, wherein the total amount of one or more viscosity reducing agents is from 4% to 7%, by weight of the hair care composition; (c) from 20% to 32% of one or more anionic surfactants, by weight of the hair care composition; and (d) from 40% to 83% of an aqueous carrier, by weight of the hair care composition; wherein the one or more viscosity reducing agents has a molecular weight of from 100 g/mol (daltons) to 300 g/mol (daltons) ; wherein the hair care composition has a liquid phase kinematic viscosity, measured at 40 degrees Celsius, of from 10 $mm^2/s$ (cSt) to 1,000 $mm^2/s$ (cSt).

Also described herein is a method of treating the hair, the method comprising (a) providing a hair care composition in a foam dispenser, wherein the hair care composition is as defined above; and (b) dispensing the hair care composition from the foam dispenser as a foam; (c) applying the foam to the hair; and (d) rinsing the foam from the hair; wherein the foam has a density of from 0.05 $g/cm^3$ to 0.30 $g/cm^3$ when dispensed from the foam dispenser.

### DETAILED DESCRIPTION OF THE INVENTION

While the specification concludes with claims particularly pointing out and distinctly claiming the invention, it is believed that the present invention will be better understood from the following description.

As used herein, the term "fluid" includes liquids and gels.

As used herein, the articles including "a" and "an" when used in a claim, are understood to mean one or more of what is claimed or described.

As used herein, "comprising" means that other steps and other ingredients which do not affect the end result can be added. This term encompasses the terms "consisting of" and "consisting essentially of".

As used herein, "mixtures" is meant to include a simple combination of materials and any compounds that may result from their combination.

As used herein, "molecular weight" or "Molecular weight" refers to the weight average molecular weight unless

otherwise stated. Molecular weight of polymers may be measured using industry standard method, gel permeation chromatography ("GPC").

As used herein, "partition dispersion coefficient" is defined by the following equation: PDC= logP - 0.3001 * $(\delta D)^2$ + 10.362 * $\delta D$ - 93.251 wherein logP is the octanol water partitioning coefficient as computed by the Consensus algorithm implemented in ACD/Percepta version 14.02 by Advanced Chemistry Development, Inc. (ACD/Labs, Toronto, Canada), and wherein $\delta D$ is the Hansen solubility dispersion parameter in $(MPa)^{1/2}$ computed using Steven Abbott and Hiroshi Yamamoto's "HSPIP - Hansen Solubility Parameters in Practice" program, $4^{th}$ Edition, version 4.1.07.

As used herein, the terms "include," "includes," and "including," are meant to be nonlimiting and are understood to mean "comprise," "comprises," and "comprising," respectively.

As used herein, the term "surfactant-soluble active" means materials which can be soluble at a concentration of 0.1% or higher in an aqueous solution of 10% sodium laureth-1 sulfate. Solubility of a material of interest can be determined by first visually assessing that the material containing sodium laureth-1 sulfate mixture is homogeneous, followed by filling a glass jar with the material containing sodium laureth-1 sulfate mixture, then placing a Class 2 standard red laser pointer such as the Quartet Class 2 standard laser pointer (model MP-1202Q) against the side of the jar and shining the laser through the jar. If the material is soluble in the sodium laureth-1 solution the laser light will not be scattered, resulting in only an observable red dot appearing on the side of the jar opposite the laser pointer and no visible red laser beam will be observed passing through the solution. The surfactant-soluble actives may be selected from the group consisting of perfumes, coloring agents, humectants, anti-dandruff agents, scalp and hair moisturizers, anti-frizz agents, anti-static agents, conditioning agents, UV filters, scalp barrier materials, styling agents, and combinations thereof.

As used herein, the term "viscosity reducing agent" means organic compounds having a molecular weight of from 100 to 300 daltons, alternatively from about 125 daltons to about 300 daltons. Additionally, the viscosity reducing agents may have a water solubility at between 23 and 25 degrees Celsius of from about 900 to 50,000 mg/L.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Unless otherwise noted, all component or composition levels are in reference to the active portion of that component or composition, and are exclusive of impurities, for example, residual solvents or by-products, which may be present in commercially available sources of such components or compositions.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

A. SURFACTANT SOLUBLE ACTIVE AGENT

The one or more surfactant soluble active agent described herein are normally insoluble or only slightly soluble within water, but can be solubilized within aqueous surfactant solutions, in alcohol or in water/alcoholic mixtures. The surfactant soluble active agent is a perfume oil and is included in an amount from 0.2% to 10%, by weight of the composition.

The surfactant soluble active agent may further comprise vitamins, UV absorbers, anti-dandruff actives, scalp benefit agents, skin actives, internal hair actives, antimicrobials, antibacterial actives, radical scavengers, skin lightening agents, chelators, antioxidants, proteins, amino acids, peptides, flavonoids, alpha-hydroxy acids, anti-acne actives, anti-inflammatories.

The total amount of the one or more surfactant soluble active agents is from 0.2% to 10% or from about 0.3% to about 5%, or from about 0.5% to about 2.5%, by weight of the composition. The surfactant soluble active agents have molecular weights ranging from 100 g/mol to 700 g/mol, or from about 125 g/mol to about 600 g/mol, or from about 150 g/mol to about 500 g/mol.

In an embodiment, surfactant soluble agents are defined as materials which are insoluble or only slightly soluble in water but soluble at a concentration of 0.1% or higher in an aqueous solution of 10% sodium laureth-1 sulfate. A conventional method may be used to determine solubility. Such method may include a method wherein solubility of a material of interest can be determined by first visually assessing that the material containing the sodium laureth-1 sulfate mixture is homogeneous, followed by filling a glass jar with the material containing sodium laureth-1 sulfate mixture, then placing a Class 2 standard red laser pointer such as the Quartet Class 2 standard laser pointer (model MP-1202Q) against 25 the side of the jar and shining the laser through the jar. If the material is soluble in the sodium laureth-1 solution the laser light will not be scattered, resulting in only an observable red dot appearing on the side of the jar

opposite the laser pointer and no visible red laser beam will be observed passing through the solution.

In an embodiment, the surfactant soluble active agent of the hair care composition described herein comprises an anti-dandruff active which may be one material or a mixture selected from the groups consisting of: azoles, such as climbazole, ketoconazole, itraconazole, econazole, and elubiol; hydroxyl pyridones, such as octopirox (piroctone olamine), ciclopirox, rilopirox, and MEA-Hydroxyoctyloxypyridinone; kerolytic agents, such as salicylic acid and other hydroxy acids; strobilurins such as azoxystrobin and metal chelators such as as 1,10-phenanthroline. In another embodiment, the azole anti-microbials is an imidazole selected from the group consisting of: benzimidazole, benzothiazole, bifonazole, butaconazole nitrate, climbazole, clotrimazole, croconazole, eberconazole, econazole, elubiol, fenticonazole, fluconazole, 10 flutimazole, isoconazole, ketoconazole, lanoconazole, metronidazole, miconazole, neticonazole, omoconazole, oxiconazole nitrate, sertaconazole, sulconazole nitrate, tioconazole, thiazole, and mixtures thereof, or the azole anti-microbials is a triazole selected from the group consisting of: terconazole, itraconazole, and mixtures thereof. In an embodiment, the azole anti-microbial active is ketoconazole. In an embodiment, the sole antimicrobial active is ketoconazole.

In the present invention, the surfactant soluble active agent is perfume which may be one material or a mixture selected from the groups: essential oils, absolutes, resinoids, resins, concretes, etc., and synthetic perfume components such as hydrocarbons, alcohols, aldehydes, ketones, ethers, acids, esters, acetals, ketals, nitriles, etc., including saturated and unsaturated compounds, aliphatic, carbocyclic and heterocyclic compounds. Examples of such perfume components are: geraniol, geranyl acetate, linalool, linalyl acetate, tetrahydrolinalool, citronellol, citronellyl acetate, dihydromyrcenol, dihydromyrcenyl acetate, tetrahydromyrcenol, terpineol, terpinyl acetate, nopol, nopyl acetate, 2-phenylethanol, 2-phenylethyl acetate, benzyl alcohol, benzyl acetate, benzyl salicylate, benzyl benzoate, styrallyl acetate, amyl salicylate, dimethylbenzylcarbinol, trichloromethylphenycarbinyl acetate, p-tert.butyl-cyclohexyl acetate, isononyl acetate, vetiveryl acetate, vetiverol, alpha-n-amylcinammic aldehyde, alpha-hexylcinammic aidehyde, 2-methyl-3-(p-tert.butylphenyl)-propanal, 2-methyl-3-(p-isopropylphenyl)propanal, 3-(ptert.butylphenyl)propanal, tricyclodecenyl acetate, tricyclodecenyl propionate, 4-(4-hydroxy-4-methylpentyl)-3-cyclohexenecarbaldehyde, 4-(4-methyl-3-pentenyl)-3-cyclohexenecarbaldehyde, 4-acetoxy-3-pentyltetrahydropyran, methyl dihydrojasmonate, 2-n-heptyl-cyclopentanone, 3-methyl-2-pentyl-cyclopentanone, n-decanal, n-dodecanal, 9-decenol-1, phenoxyethyl isobutyrate, phenylacetaldehyde dimethyl acetal, phenylacetaldehyde diethyl acetal, geranonitrile, citronellonitrile, cedryl acetate, 3-isocamphylcyclohexanol, cedryl methyl ether, isolongifolanone, aubepine nitrile, aubepine, heliotropine, coumarin, eugenol, vanillin, diphenyl oxide, hydroxycitronellal, ionones, methyl ionones, isomethyl ionones, irones, cis-3-hexenol and esters thereof, indane musks, tetralin musks, isochroman musks, macrocyclic ketones, macrolactone musks, ethylene brassylate, aromatic nitromusks.

In an embodiment, the surfactant soluble active agent comprises an anti-acne active which may be one material or a mixture selected from the groups consisting of: resorcinol, salicylic acid, benzoyl peroxide, erythromycin, and other similar materials. Other non-limiting examples of suitable anti-acne actives for use herein are described in U.S. Pat. No. 5,607,980, issued to McAtee et al.

In an embodiment, the surfactant soluble active agent comprises an anti-wrinkle active or antiatrophy active which may be one material or a mixture selected from the groups consisting of: sulfur-containing D and L amino acids and their derivatives and salts, particularly the N-acetyl derivatives, an example of which is N-acetyl-L-cysteine; thiols, e.g. ethane thiol; hydroxy acids (e.g., alpha-hydroxy acids such as lactic acid and glycolic acid or beta-hydroxy acids such as salicylic acid and salicylic acid derivatives such as the octanoyl derivative), phytic acid, lipoic acid; lysophosphatidic acid, and skin peel agents (e.g., phenol and the like). Other non-limiting examples of suitable anti-wrinkle actives for use herein are described in U.S. Pat. No. 6,217,888, issued to Oblong et al.

In an embodiment, the surfactant soluble active agent comprises an anti-oxidant or radical scavenger which may be one material or a mixture selected from the groups consisting of: ascorbic acid and its salts, ascorbyl esters of fatty acids, ascorbic acid derivatives (e.g., magnesium ascorbyl phosphate, sodium ascorbyl phosphate, ascorbyl sorbate), tocopherol, tocopherol acetate, other esters of tocopherol, butylated hydroxy benzoic acids and their salts, 6-hydroxy-2,5,7,8-tetramethylchroman-2-carboxylic acid (commercially available under the tradename Trolox®), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pilolate, nordihydroguaiaretic acid, bioflavonoids, curcumin, lysine, methionine, proline, superoxide dismutase, silymarin, tea extracts, grape skin/seed extracts, melanin, and rosemary extracts may be used.

In an embodiment, the surfactant soluble active agent comprises a flavonoid which may be one material or a mixture selected from the groups consisting of: unsubstituted flavanones, methoxy flavanones, unsubstituted chalcone, 2',4-dihydroxy chalcone, isoflavone, flavone, soy isoflavones, and mixtures thereof. Other non-limiting examples of flavanoid compounds suitable for use as skin active agents herein are described in U.S. Pat. Nos. 5,686,082 and 5,686,367

In an embodiment, the surfactant soluble active agent comprises a steroidal anti-inflammatory agent which may be one material or a mixture selected from the groups consisting of: corticosteroids such as hydrocortisone, hydroxyltriamcinolone, alpha-methyl dexamethasone, dexamethasone-phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, diflurosone diacetate, fluradrenolone acetonide, medrysone, amcinafel, amcinafide, betamethasone and the balance of its esters, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof may be used.

In an embodiment, the surfactant soluble active agent is a nonsteroidal anti-inflammatory agent which may be one material or a mixture selected from the groups consisting of: oxicams (e.g., piroxicam, isoxicam, tenoxicam, sudoxicam, CP-14,304); salicylates (e.g., aspirin, disalcid, benorylate, trilisate, safapryn, solprin, diflunisal, fendosal); acetic acid derivatives (e.g., diclofenac, fenclofenac, indomethacin, sulindac, tolmetin, isoxepac, furofenac, tiopinac, zidometacin, acematacin, fentiazac, zomepirac, clindanac, oxepinac, felbinac, ketorolac); fenamates (e.g., mefenamic, meclofenamic, flufenamic, niflumic, tolfenamic acids); propionic acid derivatives (e,g., ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, tiaprofenic); pyrazoles (e.g., phenylbutazone, oxyphenbutazone, feprazone, azapropazone, trimethazone); and combinations thereof as well as any dermatologically acceptable salts or esters of thereof.

In an embodiment, the surfactant soluble active agent comprises an antimicrobial which may be one material or a mixture selected from the groups consisting of: β-lactam drugs, quinolone drugs, ciprofloxacin, norfloxacin, tetracycline, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, ketaconazole, amanfadine hydrochloride, amanfadine sulfate, octopirox, parachlorometa xylenol, nystatin, tolnaftate, zinc pyrithione, clotrimazole, and combinations thereof.

B. VISCOSITY REDUCING AGENT

The hair care composition comprises from 4% to 7% of one or more viscosity reducing agents, by weight of the hair care composition.

The viscosity reducing agents have a partition dispersion coefficient of from 0.05 to 2.0, alternatively from about 0.08 to about 1.9, alternatively from about 0.09 to about 1.8, alternatively from about 0.095 to about 1.7, and alternatively from about 0.095 to about 1.68. The viscosity reducing agents may provide unexpected viscosity reduction and/or improved deposition of surfactant soluble active agents when used in the hair care composition described herein.

The partition dispersion coefficient (PDC) is defined by the following equation:

$$PDC = \log P - 0.3001 * (\delta D)^2 + 10.362 * \delta D - 93.251$$

wherein logP is the octanol water partitioning coefficient as computed by the Consensus algorithm implemented in ACD/Percepta version 14.02 by Advanced Chemistry Development, Inc. (ACD/Labs, Toronto, Canada), and wherein δD is the Hansen solubility dispersion parameter in $(MPa)^{1/2}$ computed using Steven Abbott and Hiroshi Yamamoto's "HSPIP - Hansen Solubility Parameters in Practice" program, $4^{th}$ Edition, version 4.1.07.

The viscosity reducing agents may be organic compounds comprising 0 polar groups, alternatively 1 polar group, alternatively at least 1 polar group, alternatively 2 to 4 polar groups, and alternative alternatively at least 2 polar groups. The polar groups may be selected from the group consisting of alcohols, aldehydes, esters, lactones, coumarins, ethers, ketones, phenol, phenyl, oxides, alkenyl, alkynyl, and combinations thereof. The polar groups may include a carbon-carbon double bond or one or more atoms selected from the group consisting of oxygen, sulfur, phosphorus,

chlorine, bromine, and combinations thereof. The viscosity reducing agents have a molecular weight of between 100 daltons and 300 daltons, alternatively from about 125 daltons to about 300 daltons. Additionally, the viscosity reducing agents may have a water solubility at between 23 and 25 degrees Celsius of from about 900 to 50,000 mg/L.

In the present invention, the viscosity reducing agents comprise limonene and are including in an amount from 4% to 7%, by weight of the composition. The viscosity reducing agents may further comprise one or more viscosity reducing agents be selected from the group consisting of veloutone, isoamyl salicylate, gamma-terpinene, linalyl iso butyrate, alpha-terpinene, dipentene, geranyl phenyl acetate, and combinations thereof.

C. COUNTERACTING ADDITIVE

The hair care composition may comprise less than 4%, alternatively less than 3%, alternatively less than 2%, alternatively less than 1% of one or more counteracting additives. The hair care composition may be substantially free of one or more counteracting additives, meaning the hair care composition comprises less than 0.5%, alternatively less than 0.3%, alternatively less than 0.1%, alternatively less than 0.05%, and alternatively less than 0.01% of one or more counteracting additives. In an embodiment, the hair care composition comprises 0% of a counteracting additive. The weight ratio of the viscosity reducing agents to the counteracting additive may be greater than 2:1, alternatively great than 3:1, alternatively greater than 4:1, alternatively greater than 5:1.

The counteracting additive may have a partition dispersion coefficient of from about -5 to about -0.7, alternatively from about -4.6 to about -0.85, alternatively from about -4.5 to about - 0.9, alternatively from about -3.1 to about -0.7, and alternatively from about -3 to about -0.85. The viscosity reducing agents may have a partition dispersion coefficient of from about -4.6 to about -1.9, alternatively from about -4.5 to about -2, wherein the one or more viscosity reducing agents has at least 2 polar groups, or has 1 polar group and less than 5 acyclic $sp^3$ hybridized carbon atoms that are connected to each other in a contiguous group. The viscosity reducing agents may have a partition dispersion coefficient of from about -4.6 to about -1.9, alternatively from about -4.5 to about -2, wherein the one or more viscosity reducing agents has 2 to 4 polar groups, or has 1 polar group and 1 to 3 acyclic $sp^3$ hybridized carbon atoms that are connected to each other in a contiguous group. The viscosity reducing agents may have a partition dispersion coefficient of from about -4.6 to about -1.9, alternatively from about -4.5 to about -2, wherein the one or more viscosity reducing agents has 2 to 4 polar groups, or has 1 polar group and 2 acyclic $sp^3$ hybridized carbon atoms that are connected to each other in a contiguous group. The one or more counteracting additives may counteract the viscosity reduction associated with the one or more viscosity reducing agents when used in the hair care composition described herein.

The partition dispersion coefficient (PDC) is defined by the following equation:

$$PDC = \log P - 0.3001 * (\delta D)^2 + 10.362 * \delta D - 93.251$$

wherein logP is the octanol water partitioning coefficient as computed by the Consensus algorithm implemented in ACD/Percepta version 14.02 by Advanced Chemistry Development, Inc. (ACD/Labs, Toronto, Canada), and wherein $\delta D$ is the Hansen solubility dispersion parameter in $(MPa)^{1/2}$ computed using Steven Abbott and Hiroshi Yamamoto's "HSPIP - Hansen Solubility Parameters in Practice"program, 4th Edition, version 4.1.07.

The counteracting additive may be an organic compound comprising 1 polar group, alternatively at least 1 polar group, alternatively 2 to 4 polar groups, and alternative alternatively at least 2 polar groups. The polar groups may be selected from the group consisting of alcohols, aldehydes, esters, lactones, coumarins, ethers, ketones, phenol, phenyl, oxides, alkenyl, alkynyl, and combinations thereof. The counteracting additive may have a molecular weight of between 100 daltons and 300 daltons, alternatively from about 125 daltons to about 300 daltons. Additionally, the counteracting additive may have a water solubility at between 23 and 25 degrees Celsius of from about 10 to 900 mg/L.

The counteracting additive may be selected from the group consisting of raspberry ketone, triethyl citrate, 5-methyl-3-heptanone oxime, hydroxycitronellal, camphor gum, 2-isopropyl-5-methyl-2-hexenal, eucalyptol, 1,1-dimethoxyoctane, isobutyl hexanoate, dihyro iso jasmonate, and combinations thereof.

The hair care composition may comprise less than 4% of one or more counteracting additives having a partition dispersion coefficient of from about 0.05 to about 5.1, by weight of the hair care composition, wherein the weight ratio of the one or more viscosity reducing agents to the counteracting additive is greater than 2:1, alternatively greater than 4:1, alternatively greater than 5:1, alternatively greater than 20:1. Alternatively, the viscosity reducing agents may be selected from the group consisting of raspberry ketone, triethyl citrate, hydroxycitronellal, camphor gum, and combinations thereof. Alternatively, the viscosity reducing agent may be selected from the group consisting of raspberry ketone, triethyl citrate, hydroxycitronellal, and combinations thereof.

D. SURFACTANTS

The hair care composition comprises from 20% to 32% alternatively from about 22% to about 28% of one or more anionic surfactants, by weight of the hair care composition.

Anionic surfactants suitable for use in the hair care composition include the alkyl and alkyl ether sulfates. Other suitable anionic surfactants are the water-soluble salts of organic, sulfuric acid reaction products. Still other suitable anionic surfactants are the reaction products of fatty acids esterified with isethionic acid and neutralized with sodium hydroxide. Other similar anionic surfactants are described in U.S. Patent Nos. 2,486,921; 2,486,922; and 2,396,278.

Exemplary anionic surfactants for use in the hair care composition include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, sodium cocoyl isethionate and combinations thereof. In a further embodiment, the anionic surfactant is sodium lauryl sulfate or sodium laureth sulfate.

In an embodiment, the hair care compositions can comprise ammonium C10-15 pareth sulfate, ammonium C10-15 alkyl sulfate, ammonium C11-15 alkyl sulfate, ammonium decyl sulfate, ammonium deceth sulfate, ammonium undecyl sulfate, ammonium undeceth sulfate, sodium C10-15 pareth sulfate, sodium C10-15 alkyl sulfate, sodium C11-15 alkyl sulfate, sodium decyl sulfate, sodium deceth sulfate, sodium undecyl sulfate, sodium undeceth sulfate, potassium C10-15 pareth sulfate, potassium C10-15 alkyl sulfate, potassium C11-15 alkyl sulfate, potassium decyl sulfate, potassium deceth sulfate, potassium undecyl sulfate, and/or potassium undeceth sulfate.

Suitable anionic surfactants include, but are not limited to undecyl sulfate compound selected from the group consisting of:

a) $R_1O(CH_2CHR_3O)_ySO_3M$;
b) $CH_3(CH_2)_zCHR_2CH_2O(CH_2CHR_3O)_ySO_3M$; and
c) mixtures thereof,

where $R_1$ represents $CH_3(CH_2)_{10}$, $R_2$ represents H or a hydrocarbon radical comprising 1 to 4 carbon atoms such that the sum of the carbon atoms in z and $R_2$ is 8, $R_3$ is H or $CH_3$, y is 0 to 7, the average value of y is about 1 when y is not zero (0), and M is a monovalent or divalent, positively-charged cation.

Suitable anionic alkyl sulfates and alkyl ether sulfate surfactants include, but are not limited to, those having branched alkyl chains which are synthesized from C8 to C18 branched alcohols which may be selected from the group consisting of: Guerbet alcohols, aldol condensation derived alcohols, oxo alcohols and mixtures thereof. Non-limiting examples of the 2-alkyl branched alcohols include oxo alcohols such as 2-methyl-1-undecanol, 2-ethyl-1-decanol, 2-propyl-1-nonanol, 2-butyl 1-octanol, 2-methyl-1-dodecanol, 2-ethyl-1-undecanol, 2-propyl-1-decanol, 2-butyl-1-nonanol, 2-pentyl-1-octanol, 2-pentyl-1-heptanol, and those sold under the tradenames LIAL® (Sasol), ISALCHEM® (Sasol), and NEODOL® (Shell), and Guerbet and aldol condensation derived alcohols such as 2-ethyl-1-hexanol, 2-propyl-1-butanol, 2-butyl-1-octanol, 2-butyl-1-decanol, 2-pentyl-1-nonanol, 2-hexyl-1-octanol, 2-hexyl-1-decanol and those sold under the tradename ISOFOL® (Sasol) or sold as alcohol ethoxylates and alkoxylates under the tradenames LUTENSOL XP® (BASF) and LUTENSOL XL® (BASF).

The anionic alkyl sulfates and alkyl ether sulfates may also include those synthesized from C8 to C18 branched alcohols derived from butylene or propylene which are sold under the trade names EXXAL™ (Exxon) and Marlipal® (Sasol). This includes anionic surfactants of the subclass of sodium trideceth-n sulfates (STnS), where n is between about 0.5 and about 3.5. Exemplary surfactants of this subclass are sodium trideceth-2 sulfates and sodium trideceth-3 sulfates. The composition of the present invention can also include sodium tridecyl sulfate.

The hair care composition may comprise from about 0.25% to about 14%, alternatively from about 1% to about 12%, alternatively from about 3% to about 10%, alternatively from about 4% to about 9% of one or more amphoteric, nonionic, or zwitterionic co-surfactants, by weight of the hair care composition. The co-surfactant can include, but is not limited to, lauramidopropyl betaine, cocoamidopropyl betaine, lauryl hydroxysultaine, sodium lauroamphoacetate, coco monoethanolamide and mixtures thereof. The hair care composition may comprise from about 2% to about 14%, alternatively from about 3% to about 10%, alternatively from about 4% to about 9% of one or more amphoteric or zwitterionic co-surfactants, by weight of the hair care composition.

Amphoteric co-surfactants suitable for use in the hair care composition include those surfactants described as derivatives of aliphatic secondary and tertiary amines in which the aliphatic radical can be straight or branched chain

and wherein one of the aliphatic substituents contains from about 8 to about 18 carbon atoms and one contains an anionic group such as carboxy, sulfonate, sulfate, phosphate, or phosphonate. Suitable amphoteric surfactant include, but are not limited to, thoseselected from the group consisting of: sodium cocaminopropionate, sodium cocaminodipropionate, sodium cocoamphoacetate, sodium cocoamphohydroxypropylsulfonate, sodium cocoamphopropionate, sodium cornamphopropionate, sodium lauraminopropionate, sodium lauroamphoacetate, sodium lauroamphohydroxypropylsulfonate, sodium lauroamphopropionate, sodium cornamphopropionate, sodium lauriminodipropionate, ammonium cocaminopropionate, ammonium cocaminodipropionate, ammonium cocoamphoacetate, ammonium cocoamphohydroxypropylsulfonate, ammonium cocoamphopropionate, ammonium cornamphopropionate, ammonium lauraminopropionate, ammonium lauroamphoacetate, ammonium lauroamphohydroxypropylsulfonate, ammonium lauroamphopropionate, ammonium cornamphopropionate, ammonium lauriminodipropionate, triethanonlamine cocaminopropionate, triethanonlamine cocaminodipropionate, triethanonlamine cocoamphoacetate, triethanonlamine cocoamphohydroxypropylsulfonate, triethanonlamine cocoamphopropionate, triethanonlamine cornamphopropionate, triethanonlamine lauraminopropionate, triethanonlamine lauroamphoacetate, triethanonlamine lauroamphohydroxypropylsulfonate, triethanonlamine lauroamphopropionate, triethanonlamine cornamphopropionate, triethanonlamine lauriminodipropionate, cocoamphodipropionic acid, disodium caproamphodiacetate, disodium caproamphoadipropionate, disodium capryloamphodiacetate, disodium capryloamphodipriopionate, disodium cocoamphocaiboxyethylhydroxypropylsulfonate, disodium cocoamphodiacetate, disodium cocoamphodipropionate, disodium dicarboxyethylcocopropylenediamine, disodium laureth-5 carboxyamphodiacetate, disodium lauriminodipropionate, disodium lauroamphodiacetate, disodium lauroamphodipropionate, disodium oleoamphodipropionate, disodium PPG-2-isodecethyl-7 carboxyamphodiacetate, lauraminopropionic acid, lauroamphodipropionic acid, lauryl aminopropylglycine, lauryl diethylenediaminoglycine, and mixtures thereof

The amphoteric co-surfactant can be a surfactant according to the following structure:

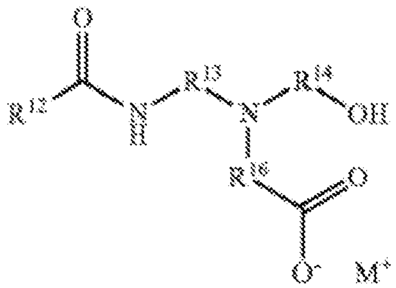

wherein R12 is a C-linked monovalent substituent selected from the group consisting of substituted alkyl systems comprising 9 to 15 carbon atoms, unsubstituted alkyl systems comprising 9 to 15 carbon atoms, straight alkyl systems comprising 9 to 15 carbon atoms, branched alkyl systems comprising 9 to 15 carbon atoms, and unsaturated alkyl systems comprising 9 to 15 carbon atoms; R13, R14, and R15 are each independently selected from the group consisting of C-linked divalent straight alkyl systems comprising 1 to 3 carbon atoms, and C-linked divalent branched alkyl systems comprising 1 to 3 carbon atoms; and M+ is a monovalent counterion selected from the group consisting of sodium, ammonium and protonated triethanolamine. In an embodiment, the amphoteric surfactant is selected from the group consisting of: sodium cocoamphoacetate, sodium cocoamphodiacetate, sodium lauroamphoacetate, sodium lauroamphodiacetate, ammonium lauroamphoacetate, ammonium cocoamphoacetate, triethanolamine lauroamphoacetate, triethanolamine cocoamphoacetate, and mixtures thereof.

The hair care composition may comprise a zwitterionic co-surfactant, wherein the zwitterionic surfactant is a derivative of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. The zwitterionic surfactant can be selected from the group consisting of: cocamidoethyl betaine, cocamidopropylamine oxide, cocamidopropyl betaine, cocamidopropyl dimethylaminohydroxypropyl hydrolyzed collagen, cocamidopropyldimonium hydroxypropyl hydrolyzed collagen, cocamidopropyl hydroxysultaine, cocobetaineamido amphopropionate, coco-betaine, cocohydroxysultaine, cocololeamidopropyl betaine, coco-sultaine, lauramidopropyl betaine, lauryl betaine, lauryl hydroxysultaine, lauryl sultaine, and mixtures thereof. A suitable zwitterionic surfactant is lauryl hydroxysultaine. The zwitterionic surfactant can be selected from the group consisting of: lauryl hydroxysultaine, cocamidopropyl hydroxysultaine, cocobetaine, coco-hydroxysultaine, coco-sultaine, lauryl betaine, lauryl sultaine, and mixtures thereof.

The co-surfactant can be a zwitterionic surfactant, wherein the zwitterionic surfactant is selected from the group consisting of: lauryl hydroxysultaine, cocamidopropyl hydroxysultaine, coco-betaine, coco-hydroxysultaine, cocosultaine, lauryl betaine, lauryl sultaine, and mixtures thereof.

In an embodiment, the co-surfactant is selected from amphoteric or zwitterionic surfactants synthesized from lauric acid including, but not limited to, lauramidopropyl betaine, lauryl Hydroxysultaine, and sodium lauroamphoacetate and having a chain length distribution wherein the C12 chain length averages from about 80% to about 100%, alternatively from about 85% to about 100%, alternatively from about 90% to about 100%, alternatively from about 95% to about 100%, and alternatively from about 97% to about 100% of the total molecular chain length distribution.

Suitable nonionic surfactants for use in the hair care composition include those described in McCutcheion's Detergents and Emulsifiers, North American edition (1986), Allured Publishing Corp., and McCutcheion's Functional Materials, North American edition (1992). Suitable nonionic surfactants for use in the hair care composition include, but are not limited to, polyoxyethylenated alkyl phenols, polyoxyethylenated alcohols, polyoxyethylenated polyoxypropylene glycols, glyceryl esters of alkanoic acids, polyglyceryl esters of alkanoic acids, propylene glycol esters of alkanoic acids, sorbitol esters of alkanoic acids, polyoxyethylenated sorbitor esters of alkanoic acids, polyoxyethylene glycol esters of alkanoic acids, polyoxyethylenated alkanoic acids, alkanolamides, N-alkylpyrrolidones, alkyl glycosides, alkyl polyglucosides, alkylamine oxides, and polyoxyethylenated silicones.

The non-ionic surfactant may be selected from the group consisting of: Cocamide, Cocamide Methyl MEA, Cocamide DEA, Cocamide MEA, Cocamide MIPA, Lauramide DEA, Lauramide MEA, Lauramide MIPA, Myristamide DEA, Myristamide MEA, PEG-20 Cocamide MEA, PEG-2 Cocamide, PEG-3 Cocamide, PEG-4 Cocamide, PEG-5 Cocamide, PEG-6 Cocamide, PEG-7 Cocamide, PEG-3 Lauramide, PEG-5 Lauramide, PEG-3 Oleamide, PPG-2 Cocamide, PPG-2 Hydroxyethyl Cocamide, and mixtures thereof.

Non limiting examples of other anionic, zwitterionic, non-ionic, and amphoteric additional surfactants suitable for use in the hair care composition are described in McCutcheon's, Emulsifiers and Detergents, 1989 Annual, published by M. C. Publishing Co., and U.S. Patent Nos. 3,929,678, 2,658,072; 2,438,091; 2,528,378,

E. CATIONIC POLYMERS

The hair care composition described herein may also comprise one or more cationic polymers. These cationic polymers may be selected from the group consisting of cationic guar polymers, cationic non-guar galactomannan polymers, cationic tapioca polymers, cationic copolymers of acrylamide monomers and cationic monomers, synthetic noncrosslinked cationic polymers which may or may not form lyotropic liquid crystals upon combination with the detersive surfactant, cationic cellulose polymers, and combinations thereof. The hair care composition may comprise a cationic polymer selected from the group consisting of guar polymers, non-guar galactomannan polymers, tapioca polymers, copolymers of acrylamide monomers and cationic monomers, cellulose polymers, and combinations thereof.

The hair care composition may comprise a cationic guar polymer, which is a cationically substituted galactomannan (guar) gum derivatives. Guar gum for use in preparing these guar gum derivatives may be obtained as a naturally occurring material from the seeds of the guar plant. The guar molecule itself is a straight chain mannan, which is branched at regular intervals with single membered galactose units on alternative mannose units. The mannose units are linked to each other by means of $\beta$(1-4) glycosidic linkages. The galactose branching arises by way of an $\alpha$(1-6) linkage. Cationic derivatives of the guar gums are obtained by reaction between the hydroxyl groups of the polygalactomannan and reactive quaternary ammonium compounds. The degree of substitution of the cationic groups onto the guar structure should be sufficient to provide the requisite cationic charge density described above.

The cationic polymer, including but not limited to a cationic guar polymer, may have a molecular weight of less than 1.0 million g/mol, or from about 10 thousand to about 1 million g/mol, or from about 25 thousand to about 1million g/mol, or from about 50 thousand to about 1 million g/mol, or from about 100 thousand to about 1 million g/mol. In one embodiment, the cationic guar polymer has a charge density of from about 0.2 to about 2.2 meq/g, or from about 0.3 to about 2.0 meq/g, or from about 0.4 to about 1.8 meq/g; or from about 0.5 meq/g to about 1.7 meq/g.

The cationic guar polymer may have a weight average molecular weight of less than about 1.0 million g/mol, and has a charge density of about 0.1 meq/g to about 2.5 meq/g. In an embodiment, the cationic guar polymer has a weight average molecular weight of less than 950 thousand g/mol, or from about 10 thousand to about 900 thousand g/mol, or from about 25 thousand to about 900 thousand g/mol, or from about 50 thousand to about 900 thousand g/mol, or from about 100 thousand to about 900 thousand g/mol. from about 150 thousand to about 800 thousand g/mol. Alternatively, the cationic guar polymer may have a charge density of from about 0.2 to about 2.2 meq/g, or from about 0.3 to about 2.0 meq/g, or from about 0.4 to about 1.8 meq/g, or from about 0.5 meq/g to about 1.5 meq/g.

The hair care composition can comprise from about 0.05% to less than about 1%, from about 0.05% to about 0.9%, from about 0.1% to about 0.8%, or from about 0.2% to about 0.7% of the one or more cationic polymers, by weight of the hair care composition.

The cationic guar polymer may be formed from quaternary ammonium compounds. In an embodiment, the quaternary ammonium compounds for forming the cationic guar polymer conform to the general formula 1:

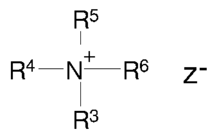

wherein where $R^3$, $R^4$ and $R^5$ are methyl or ethyl groups; $R^6$ is either an epoxyalkyl group of the general formula 2:

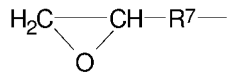

or $R^6$ is a halohydrin group of the general formula 3:

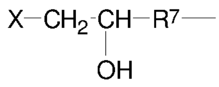

wherein $R^7$ is a $C_1$ to $C_3$ alkylene; X is chlorine or bromine, and Z is an anion such as Cl-, Br-, Ior $HSO_4$-.

In an embodiment, the cationic guar polymer conforms to the general formula 4:

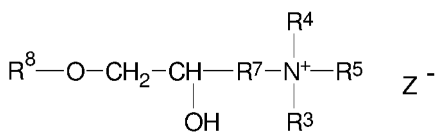

wherein $R^8$ is guar gum; and wherein $R^4$, $R^5$, $R^6$ and $R^7$ are as defined above; and wherein Z is a halogen. In an embodiment, the cationic guar polymer conforms to formula 5:

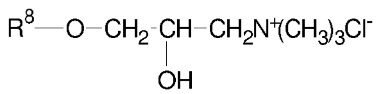

Suitable cationic guar polymers include cationic guar gum derivatives, such as guar hydroxypropyltrimonium chloride. The cationic guar polymer may be a guar hydroxypropyltrimonium chloride. Examples of guar hydroxypropyltrimonium chlorides include the Jaguar® series commercially available from Solvay, for example Jaguar® C-500, commercially available from Solvay. Jaguar® C-500 has a charge density of 0.8 meq/g and a molecular weight of 500,000 g/mol. Other suitable guar hydroxypropyltrimonium chloride include guar hydroxypropyltrimonium chloride which has a charge density of about 1.1 meq/g and a molecular weight of about 500,000 g/mol is available from ASI, a charge density of about 1.5 meq/g and a molecular weight of about 500,000 g/mole is available from ASI. Other suitable guar hydroxypropyltrimonium chloride include Hi-Care 1000, which has a charge density of about 0.7 meq/g and a molecular weight of about 600,000 g/mole and is available from Rhodia; N-Hance 3269 and N-Hance 3270, which has a charge density of about 0.7 meq/g and a molecular weight of about 425,000 g/mol and is available from ASIAquaCat CG518, has a charge density of about 0.9 meq/g, a molecular weightof about 50,000 g/mol, and is available from ASI. BF-13, which is a borate (boron) free guar of charge density of about 1.1 meq/g and molecular weight of about 800,000 and BF-17, which is a borate (boron) free guar of charge density of about 1.7 meq/g and M. W.t of about 800,000 both available from ASI.

The hair care compositions described herein may comprise a galactomannan polymer derivative having a mannose to galactose ratio of greater than 2:1 on a monomer to monomer basis, the galactomannan polymer derivative selected from the group consisting of a cationic galactomannan polymer derivative and an amphoteric galactomannan polymer derivative having a net positive charge. As used herein, the term "cationic galactomannan" refers to a galactomannan polymer to which a cationic group is added. The term "amphoteric galactomannan" refers to a galactomannan polymer to which a cationic group and an anionic group are added such that the polymer has a net positive charge.

Galactomannan polymers are present in the endosperm of seeds of the Leguminosae family. Galactomannan polymers are made up of a combination of mannose monomers and galactose monomers. The galactomannan molecule is a straight chain mannan branched at regular intervals with single membered galactose units on specific mannose units. The mannose units are linked to each other by means of β (1-4) glycosidic linkages. The galactose branching

arises by way of an α (1-6) linkage. The ratio of mannose monomers to galactose monomers varies according to the species of the plant and also is affected by climate. Non Guar Galactomannan polymer derivatives of the present invention have a ratio of mannose to galactose of greater than 2:1 on a monomer to monomer basis. Suitable ratios of mannose to galactose can be greater than about 3:1, and the ratio of mannose to galactose can be greater than about 4:1. Analysis of mannose to galactose ratios is well known in the art and is typically based on the measurement of the galactose content.

The gum for use in preparing the non-guar galactomannan polymer derivatives is typically obtained as naturally occurring material such as seeds or beans from plants. Examples of various non-guar galactomannan polymers include but are not limited to Tara gum (3 parts mannose/1 part galactose), Locust bean or Carob (4 parts mannose/1 part galactose), and Cassia gum (5 parts mannose/1 part galactose).

The non-guar galactomannan polymer derivatives may have a molecular weight from about 1,000 to about 10,000,000, alternatively from about 5,000 to about 3,000,000, alternatively from about 1,000 to about1,000,000, and alternatively from about 5,000 to about 900,000.

The hair care compositions may also include galactomannan polymer derivatives which have a cationic charge density from about 0.5 meq/g to about 7 meq/g. The galactomannan polymer derivatives may have a cationic charge density from about 1 meq/g to about 5 meq/g. The degree of substitution of the cationic groups onto the galactomannan structure should be sufficient to provide the requisite cationic charge density.

The galactomannan polymer derivative can be a cationic derivative of the non-guar galactomannan polymer, which is obtained by reaction between the hydroxyl groups of the polygalactomannan polymer and reactive quaternary ammonium compounds. Suitable quaternary ammonium compounds for use in forming the cationic galactomannan polymer derivatives include those conforming to the general formulas 1-5, as defined above.

Cationic non-guar galactomannan polymer derivatives formed from the reagents described above are represented by the general formula 6:

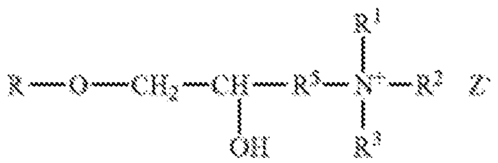

wherein R is the gum. The cationic galactomannan derivative can be a gum hydroxypropyltrimethylammonium chloride, which can be more specifically represented by the general formula 7:

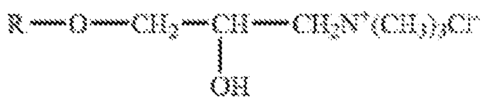

Alternatively the galactomannan polymer derivative can be an amphoteric galactomannan polymer derivative having a net positive charge, obtained when the cationic galactomannan polymer derivative further comprises an anionic group.

The cationic non-guar galactomannan can have a ratio of mannose to galactose is greater than about 4:1, a molecular weight of about 50,000g/mol to about 1,000,000g/mol, and/or from about 100,000 g/mol to about 900,000 g/mol and a cationic charge density from about 1 meq/g to about 5 meq/g, and/or from 2 meq/ g to about 4 meq/ g and can also be derived from a cassia plant.

The hair care compositions may comprise at least about 0.05% of a galactomannan polymer derivative by weight of the composition, alternatively from about 0.05% to about 2%, by weight of the composition, of a galactomannan polymer derivative.

The hair care compositions may comprise water-soluble cationically modified starch polymers. As used herein, the term "cationically modified starch" refers to a starch to which a cationic group is added prior to degradation of the starch to a smaller molecular weight, or wherein a cationic group is added after modification of the starch to achieve a desired molecular weight. The definition of the term "cationically modified starch" also includes amphoterically modified starch. The term "amphoterically modified starch" refers to a starch hydrolysate to which a cationic group and an anionic group are added.

The hair care compositions may comprise cationically modified starch polymers at a range of about 0.01% to about 10%, and/or from about 0.05% to about 5%, by weight of the composition.

The cationically modified starch polymers disclosed herein have a percent of bound nitrogen of from about 0.5% to about 4%.

The cationically modified starch polymers for use in the hair care compositions can have a molecular weight about 50,000 g/mol to about 1,000,000 g/mol and/or from about 100,000 g/mol to about 1,000,000 g/mol.

The hair care compositions may include cationically modified starch polymers which have a charge density of from about 0.2 meq/g to about 5 meq/g, and/or from about 0.2 meq/g to about 2 meq/g. The chemical modification to obtain such a charge density includes, but is not limited to, the addition of amino and/or ammonium groups into the starch molecules. Nonlimiting examples of these ammonium groups may include substituents such as hydroxypropyl trimmonium chloride, trimethylhydroxypropyl ammonium chloride, dimethylstearylhydroxypropyl ammonium chloride, and dimethyldodecylhydroxypropyl ammonium chloride. See Solarek, D. B., Cationic Starches in Modified Starches: Properties and Uses, Wurzburg, O. B., Ed., CRC Press, Inc., Boca Raton, Fla. 1986, pp 113-125. The cationic groups may be added to the starch prior to degradation to a smaller molecular weight or the cationic groups may be added after such modification.

The cationically modified starch polymers generally have a degree of substitution of a cationic group from about 0.2 to about 2.5. As used herein, the "degree of substitution" of the cationically modified starch polymers is an average measure of the number of hydroxyl groups on each anhydroglucose unit which is derivatized by substituent groups. Since each anhydroglucose unit has three potential hydroxyl groups available for substitution, the maximum possible degree of substitution is 3. The degree of substitution is expressed as the number of moles of substituent groups per mole of anhydroglucose unit, on a molar average basis. The degree of substitution may be determined using proton nuclear magnetic resonance spectroscopy (".sup.1H NMR") methods well known in the art. Suitable .sup.1H NMR techniques include those described in "Observation on NMR Spectra of Starches in Dimethyl Sulfoxide, Iodine-Complexing, and Solvating in Water-Dimethyl Sulfoxide", Qin-Ji Peng and Arthur S. Perlin, Carbohydrate Research, 160 (1987), 57-72; and "An Approach to the Structural Analysis of Oligosaccharides by NMR Spectroscopy", J. Howard Bradbury and J. Grant Collins, Carbohydrate Research, 71, (1979), 15-25.

The source of starch before chemical modification can be chosen from a variety of sources such as tubers, legumes, cereal, and grains. Non-limiting examples of this source starch may include corn starch, wheat starch, rice starch, waxy corn starch, oat starch, cassaya starch, waxy barley, waxy rice starch, glutenous rice starch, sweet rice starch, amioca, potato starch, tapioca starch, oat starch, sago starch, sweet rice, or mixtures thereof.

The cationically modified starch polymers can be selected from degraded cationic maize starch, cationic tapioca, cationic potato starch, and mixtures thereof. Alternatively, the cationically modified starch polymers are cationic corn starch and cationic tapioca.

The starch, prior to degradation or after modification to a smaller molecular weight, may comprise one or more additional modifications. For example, these modifications may include cross-linking, stabilization reactions, phosphorylations, and hydrolyzations. Stabilization reactions may include alkylation and esterification.

The cationically modified starch polymers may be incorporated into the composition in the form of hydrolyzed starch (e.g., acid, enzyme, or alkaline degradation), oxidized starch (e.g., peroxide, peracid, hypochlorite, alkaline, or any other oxidizing agent), physically/mechanically degraded starch (e.g., via the thermo-mechanical energy input of the processing equipment), or combinations thereof.

An optimal form of the starch is one which is readily soluble in water and forms a substantially clear (% Transmittance.gtoreq.80 at 600 nm) solution in water. The transparency of the composition is measured by Ultra-Violet/Visible (UV/VIS) spectrophotometry, which determines the absorption or transmission of UV/VIS light by a sample, using a Gretag Macbeth Colorimeter Color i 5 according to the related instructions. A light wavelength of 600 nm has been shown to be adequate for characterizing the degree of clarity of cosmetic compositions.

Also suitable for use in the hair care composition described herein are nonionic modified starches that can be further derivatized to a cationically modified starch as is known in the art. Other suitable modified starch starting materials may be quaternized to produce the cationically modified starch polymer suitable for use in hair care compositions.

Starch Degradation Procedure: a starch slurry can be prepared by mixing granular starch in water. The temperature is raised to about 35°C. An aqueous solution of potassium permanganate is then added at a concentration of about 50 ppm based on starch. The pH is raised to about 11.5 with sodium hydroxide and the slurry is stirred sufficiently to prevent settling of the starch. Then, about a 30% solution of hydrogen peroxide diluted in water is added to a level of about 1% of peroxide based on starch. The pH of about 11.5 is then restored by adding additional sodium hydroxide. The reaction is completed over about a 1 to about 20 hour period. The mixture is then neutralized with dilute hydrochloric acid. The degraded starch is recovered by filtration followed by washing and drying.

The hair care composition can comprise a cationic copolymer of an acrylamide monomer and a cationic monomer, wherein the copolymer has a charge density of from about 1.0 meq/g to about 3.0 meq/g. The cationic copolymer can be a synthetic cationic copolymer of acrylamide monomers and cationic monomers.

The cationic copolymer can comprise:

(i) an acrylamide monomer of the following Formula AM:

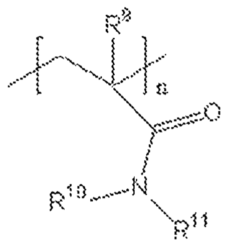

Formula AM

where $R^9$ is H or $C_{1-4}$ alkyl; and $R^{10}$ and $R^{11}$ are independently selected from the group consisting of H, $C_{1-4}$ alkyl, $CH_2OCH_3$, $CH_2OCH_2CH(CH_3)_2$, and phenyl, or together are $C_{3-6}$cycloalkyl; and

(ii) a cationic monomer conforming to Formula CM:

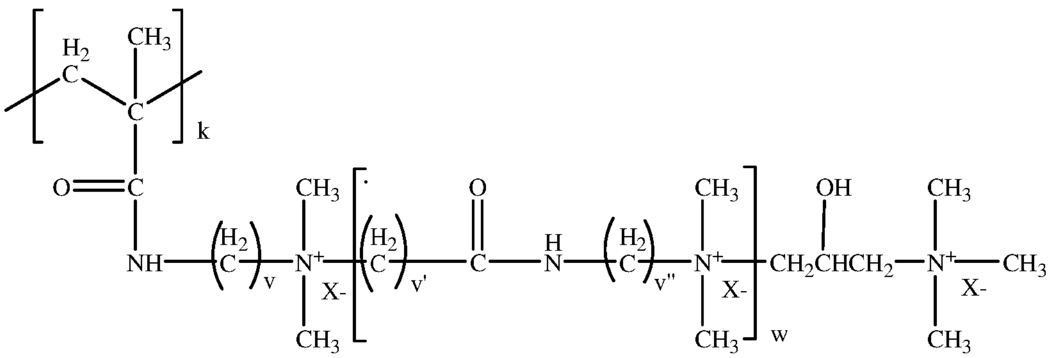

Formula CM

where k = 1, each of v, v', and v" is independently an integer of from 1 to 6, w is zero or an integer of from 1 to 10, and $X^-$ is an anion.

The cationic monomer can conform to Formula CM and where k = 1, v = 3 and w = 0, z = 1 and $X^-$ is $Cl^-$ to form the following structure:

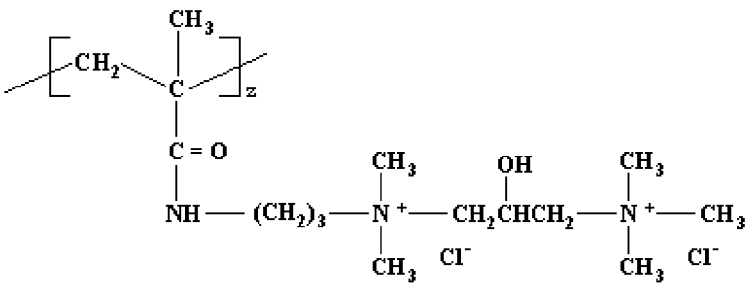

The above structure may be referred to as diquat. Alternatively, the cationic monomer can conform to Formula CM and wherein v and v" are each 3, v' = 1, w =1, y = 1 and $X^-$ is $Cl^-$, such as:

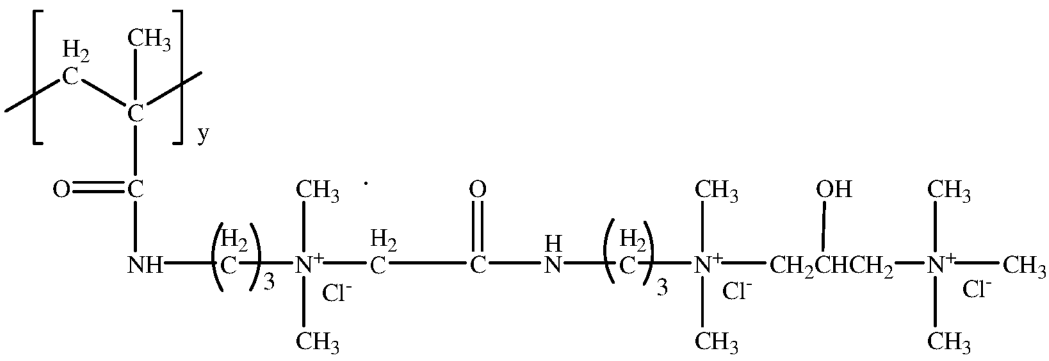

The above structure may be referred to as triquat.

Suitable acrylamide monomer include, but are not limited to, either acrylamide or methacrylamide.

In an alternative embodiment, the cationic copolymer is of an acrylamide monomer and a cationic monomer, wherein the cationic monomer is selected from the group consisting of: dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide; ethylenimine, vinylamine, 2-vinylpyridine, 4-vinylpyridine; trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride, and mixtures thereof.

The cationic copolymer can comprise a cationic monomer selected from the group consisting of: cationic monomers include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, and mixtures thereof.

The cationic copolymer can be water-soluble. The cationic copolymer is formed from (1) copolymers of (meth)acrylamide and cationic monomers based on (meth)acrylamide, and/or hydrolysis-stable cationic monomers, (2) terpolymers of (meth)acrylamide, monomers based on cationic (meth)acrylic acid esters, and monomers based on (meth)acrylamide, and/or hydrolysis-stable cationic monomers. Monomers based on cationic (meth)acrylic acid esters may be cationized esters of the (meth)acrylic acid containing a quaternized N atom. In an embodiment, cationized esters of the (meth)acrylic acid containing a quaternized N atom are quaternized dialkylaminoalkyl (meth)acrylates with C1 to C3 in the alkyl and alkylene groups. Suitable cationized esters of the (meth)acrylic acid containing a quaternized N atom can be selected from the group consisting of: ammonium salts of dimethylaminomethyl (meth)acrylate, dimethylaminoethyl (meth)acrylate, dimethylaminopropyl (meth)acrylate, diethylaminomethyl (meth)acrylate, diethylaminoethyl (meth)acrylate; and diethylaminopropyl (meth)acrylate quaternized with methyl chloride. In an embodiment, the cationized esters of the (meth)acrylic acid containing a quaternized N atom is dimethylaminoethyl acrylate, which is quaternized with an alkyl halide, or with methyl chloride or benzyl chloride or dimethyl sulfate (ADAME-Quat). the cationic monomer when based on (meth)acrylamides can be quaternized dialkylaminoalkyl(meth)acrylamides with C1 to C3 in the alkyl and alkylene groups, or dimethylaminopropylacrylamide, which is quaternized with an alkyl halide, or methyl chloride or benzyl chloride or dimethyl sulfate.

Suitable cationic monomer based on a (meth)acrylamide include quaternized dialkylaminoalkyl(meth)acrylamide with C1 to C3 in the alkyl and alkylene groups. The cationic monomer based on a (meth)acrylamide can be dimethylaminopropylacrylamide, which is quaternized with an alkyl halide, especially methyl chloride or benzyl chloride or dimethyl sulfate.

The cationic monomer can be a hydrolysis-stable cationic monomer. Hydrolysis-stable cationic monomers can be, in addition to a dialkylaminoalkyl(meth)acrylamide, all monomers that can be regarded as stable to the OECD hydrolysis test. The cationic monomer can be hydrolysis-stable and the hydrolysis-stable cationic monomer can be selected from the group consisting of: diallyldimethylammonium chloride and water-soluble, cationic styrene derivatives.

The cationic copolymer can be a terpolymer of acrylamide, 2-dimethylammoniumethyl (meth)acrylate quaternized with methyl chloride (ADAME-Q) and 3-dimethylammoniumpropyl(meth)acrylamide quaternized with methyl chloride (DIMAPA-Q). The cationic copolymer can be formed from acrylamide and acrylamidopropyltrimethylammonium chloride, wherein the acrylamidopropyltrimethylammonium chloride has a charge density of from about 1.0 meq/g to about 3.0 meq/g.

The cationic copolymer can have a charge density of from about 1.1 meq/g to about 2.5 meq/g, or from about 1.1 meq/g to about 2.3 meq/g, or from about 1.2 meq/g to about 2.2 meq/g, or from about 1.2 meq/g to about 2.1 meq/g,

or from about 1.3 meq/g to about 2.0 meq/g, or from about 1.3 meq/g to about 1.9 meq/g.

The cationic copolymer can have a molecular weight from about 10 thousand g/mol to about 1 million g/mol, or from about 25 thousand g/mol to about 1 million g/mol, or from about 50 thousand g/mol to about 1 million g/mol, or from about 100 thousand g/mol to about 1.0 million g/mol, or from about 150 thousand g/mol to about 1.0 million g/mol.

(a) Cationic Synthetic Polymers

The hair care composition can comprise a cationic synthetic polymer that may be formed from

i) one or more cationic monomer units, and optionally
ii) one or more monomer units bearing a negative charge, and/or
iii) a nonionic monomer,

wherein the subsequent charge of the copolymer is positive. The ratio of the three types of monomers is given by "m", "p" and "q" where "m" is the number of cationic monomers, "p" is the number of monomers bearing a negative charge and "q" is the number of nonionic monomers

The cationic polymers can be water soluble or dispersible, non-crosslinked, and synthetic cationic polymers having the following structure:

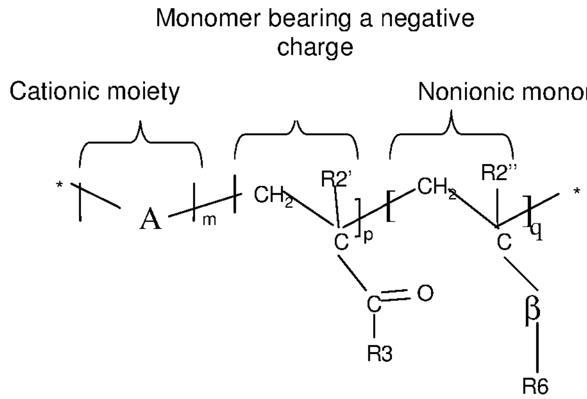


$m \geq 1$
$p=0$ or $\geq 1$
$q=0$ or $\geq 1$
$m \geq p$

where A, may be one or more of the following cationic moieties:

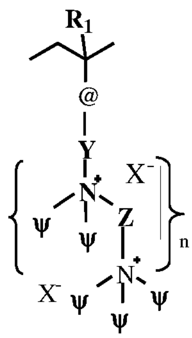
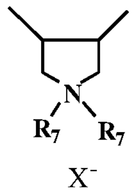
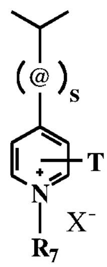

where @ = amido, alkylamido, ester, ether, alkyl or alkylaryl;
where Y = C1-C22 alkyl, alkoxy, alkylidene, alkyl or aryloxy;
where ψ = C1-C22 alkyl, alkyloxy, alkyl aryl or alkyl arylox;.
where Z = C1-C22 alkyl, alkyloxy, aryl or aryloxy;
where R1 = H, C1-C4 linear or branched alkyl;
where s = 0 or1, n = 0 or≥ 1;
where T and R7 = C1-C22 alkyl; and
where X- = halogen, hydroxide, alkoxide, sulfate or alkylsulfate.

Where the monomer bearing a negative charge is defined by R2' = H, C1-C4 linear or branched alkyl and R3 as:

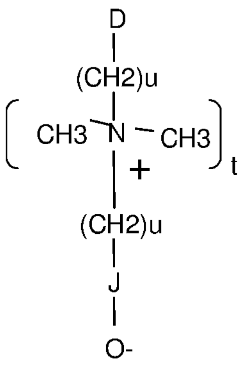 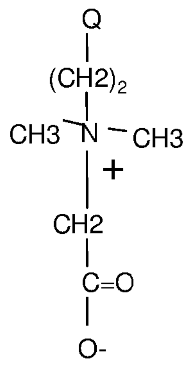 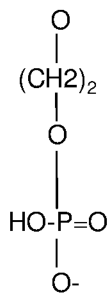 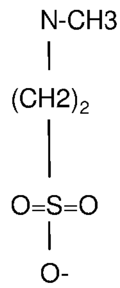

where D = O, N, or S;
where Q = $NH_2$ or O;
where u = 1-6;
where t = 0-1; and
where J = oxygenated functional group containing the following elements P, S, C.

Where the nonionic monomer is defined by R2" = H, C1-C4 linear or branched alkyl, R6 = linear or branched alkyl, alkyl aryl, aryl oxy, alkyloxy, alkylaryl oxy and β is defined as

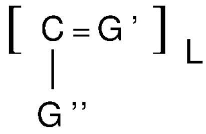

;

and
where G' and G" are, independently of one another, O, S or N-H and L =0 or 1.

Examples of cationic monomers include aminoalkyl (meth)acrylates, (meth)aminoalkyl (meth)acrylamides; monomers comprising at least one secondary, tertiary or quaternary amine function, or a heterocyclic group containing a nitrogen atom, vinylamine or ethylenimine; diallyldialkyl ammonium salts; their mixtures, their salts, and macromonomers deriving from therefrom.

Further examples of cationic monomers include dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide, ethylenimine, vinylamine, 2-vinylpyridine, 4- vinylpyridine, trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride.

Suitable cationic monomers include those which comprise a quaternary ammonium group of formula $-NR_3^+$, wherein R, which is identical or different, represents a hydrogen atom, an alkyl group comprising 1 to 10 carbon atoms, or a benzyl group, optionally carrying a hydroxyl group, and comprise an anion (counter-ion). Examples of anions are halides such as chlorides, bromides, sulphates, hydrosulphates, alkylsulphates (for example comprising 1 to 6 carbon atoms), phosphates, citrates, formates, and acetates.

Suitable cationic monomers include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride.

Additional suitable cationic monomers include trimethyl ammonium propyl (meth)acrylamido chloride.

Examples of monomers bearing a negative charge include alpha ethylenically unsaturated monomers comprising a phosphate or phosphonate group, alpha ethylenically unsaturated monocarboxylic acids, monoalkylesters of alpha ethylenically unsaturated dicarboxylic acids, monoalkylamides of alpha ethylenically unsaturated dicarboxylic acids, alpha ethylenically unsaturated compounds comprising a sulphonic acid group, and salts of alpha ethylenically unsaturated compounds comprising a sulphonic acid group.

Suitable monomers with a negative charge include acrylic acid, methacrylic acid, vinyl sulphonic acid, salts of vinyl sulfonic acid, vinylbenzene sulphonic acid, salts of vinylbenzene sulphonic acid, alpha-acrylamidomethylpropanesulphonic acid, salts of alpha-acrylamidomethylpropanesulphonic acid, 2-sulphoethyl methacrylate, salts of 2-sulphoethyl methacrylate, acrylamido-2-methylpropanesulphonic acid (AMPS), salts of acrylamido-2-methylpropanesulphonic acid, and styrenesulphonate (SS).

Examples of nonionic monomers include vinyl acetate, amides of alpha ethylenically unsaturated carboxylic acids, esters of an alpha ethylenically unsaturated monocarboxylic acids with an hydrogenated or fluorinated alcohol, polyethylene oxide (meth)acrylate (i.e. polyethoxylated (meth)acrylic acid), monoalkylesters of alpha ethylenically unsaturated dicarboxylic acids, monoalkylamides of alpha ethylenically unsaturated dicarboxylic acids, vinyl nitriles, vinylamine amides, vinyl alcohol, vinyl pyrolidone, and vinyl aromatic compounds.

Suitable nonionic monomers include styrene, acrylamide, methacrylamide, acrylonitrile, methylacrylate, ethylacrylate, n-propylacrylate, n-butylacrylate, methylmethacrylate, ethylmethacrylate, n-propylmethacrylate, n-butylmethacrylate, 2-ethyl-hexyl acrylate, 2-ethyl-hexyl methacrylate, 2-hydroxyethylacrylate and 2-hydroxyethylmethacrylate.

The anionic counterion (X-) in association with the synthetic cationic polymers may be any known counterion so long as the polymers remain soluble or dispersible in water, in the hair care composition, or in a coacervate phase of the hair care composition, and so long as the counterions are physically and chemically compatible with the essential components of the hair care composition or do not otherwise unduly impair product performance, stability or aesthetics. Non limiting examples of such counterions include halides (e.g., chlorine, fluorine, bromine, iodine), sulfate and methylsulfate.

The concentration of the cationic polymers ranges about 0.025% to about 5%, from about 0.1% to about 3%, and/or from about 0.2% to about 1%, by weight of the hair care composition.

Suitable cationic cellulose polymers are salts of hydroxyethyl cellulose reacted with trimethyl ammonium substituted epoxide, referred to in the industry (CTFA) as Polyquaternium 10 and available from Dow/ Amerchol Corp. (Edison, N.J., USA) in their Polymer LR, JR, and KG series of polymers. Other suitable types of cationic cellulose include the polymeric quaternary ammonium salts of hydroxyethyl cellulose reacted with lauryl dimethyl ammonium-substituted epoxide referred to in the industry (CTFA) as Polyquaternium 24. These materials are available from Dow/ Amerchol Corp. under the tradename Polymer LM-200. Other suitable types of cationic cellulose include the polymeric quaternary ammonium salts of hydroxyethyl cellulose reacted with lauryl dimethyl ammonium-substituted epoxide and trimethyl ammonium substituted epoxide referred to in the industry (CTFA) as Polyquaternium 67. These materials are available from Dow/ Amerchol Corp. under the tradename SoftCAT Polymer SL-5, SoftCAT Polymer SL-30, Polymer SL-60, Polymer SL-100, Polymer SK-L, Polymer SK-M, Polymer SK-MH, and Polymer SK-H.

## F. PROPELLANT

The hair care composition described herein may comprise from about 1% to about 10% propellant, alternatively from about 2% to about 8% propellant, and alternatively from about 2.5% to about 7% propellant, by weight of the hair care composition.

The propellant may comprise one or more volatile materials, which in a gaseous state, may carry the other components of the hair care composition in particulate or droplet form. The propellant may have a boiling point within the range of from about -45° C. to about 5° C. The propellant may be liquefied when packaged in convention aerosol containers under pressure. The rapid boiling of the propellant upon leaving the aerosol foam dispenser may aid in the atomization of the other components of the hair care composition.

Aerosol propellants which may be employed in the hair care composition may include the chemically-inert hydrocarbons such as propane, n-butane, isobutane, cyclopropane, and mixtures thereof, as well as halogenated hydrocarbons such as dichlorodifluoromethane, 1,1-dichloro-1,1,2,2-tetrafluoroethane, 1-chloro-1-difluoro-2,2-trifluoroethane, 1-chloro-1,1-difluoroethylene, 1,1-difluoroethane, dimethyl ether, monochlorodifluoromethane, trans-1,3,3,3-tetrafluoropropene, and mixtures thereof. The propellant may comprise hydrocarbons such as isobutane, propane, and butane-these materials may be used for their low ozone reactivity and may be used as individual components where their vapor pressures at 21.1° C. range from about 1.17 Bar to about 7.45 Bar, alternatively from about 1.17 Bar to about 4.83 Bar, and alternatively from about 2.14 Bar to about 3.79 Bar.

## G. OPTIONAL INGREDIENTS

The hair care composition described herein may further comprise one or more optional ingredients, including benefit agents Suitable benefit agents include, but are not limited to conditioning agents, cationic polymers silicone emulsions, anti-dandruff actives, gel networks, chelating agents, and natural oils such as sun flower oil or castor oil. Additional suitable optional ingredients include but are not limited to perfumes, perfume microcapsules, colorants, particles, anti-microbials, foam busters, anti-static agents, rheology modifiers and thickeners, suspension materials and structurants, pH adjusting agents and buffers, preservatives, pearlescent agents, solvents, diluents, anti-oxidants, vitamins and combinations thereof.

Such optional ingredients should be physically and chemically compatible with the components of the composition, and should not otherwise unduly impair product stability, aesthetics, or performance. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the composition herein.

1. Conditioning Agents

The conditioning agent may be a silicone conditioning agent. The silicone conditioning agent may comprise volatile silicone, non-volatile silicone, or combinations thereof. The concentration of the silicone conditioning agent may range from about 0.01% to about 10%, by weight of the composition, from about 0.1% to about 8%, from about 0.1% to about 5%, and/or from about 0.2% to about 3%. Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609.

The silicone conditioning agents for use herein may have a viscosity, as measured at 25° C, from about 20 to about 2,000,000 centistokes ("csk"), from about 1,000 to about 1,800,000 csk, from about 10,000 to about 1,500,000 csk, and/or from about 20,000 to about 1,500,000 csk.

The dispersed silicone conditioning agent particles may have a volume average particle diameter ranging from about 0.01 micrometer to about 60 micrometer. For small particle application to hair, the volume average particle diameters typically range from about 0.01 micrometer to about 4 micrometer, from about 0.01 micrometer to about 2 micrometer, from about 0.01 micrometer to about 0.5 micrometer.

Additional material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989), incorporated herein by reference.

Silicone emulsions suitable for use in the hair care composition described herein include, but are not limited to, emulsions of insoluble polysiloxanes prepared in accordance with the descriptions provided in U.S. Patent No. 4,476,282 and U.S. Patent Application Publication No. 2007/0276087. Accordingly, suitable insoluble polysiloxanes include polysiloxanes such as alpha, omega hydroxy-terminated polysiloxanes or alpha, omega alkoxy-terminated polysiloxanes having a molecular weight within the range from about 50,000 to about 500,000 g/mol. In an embodiment, the insoluble polysiloxane can have an average molecular weight within the range from about 50,000 to about 500,000 g/mol. For example, the insoluble polysiloxane may have an average molecular weight within the range from about 60,000 to about 400,000; from about 75,000 to about 300,000; from about 100,000 to about 200,000; or the average molecular weight may be about 150,000 g/mol. In an embodiment, the insoluble polysiloxane can have an internal phase viscosity of from about 5 centistokes to about 500,000 centistokes. The insoluble polysiloxane can have an average particle size within the range from about 10 nm to about 10 micron or from about 30 nm to about 10 micron. The average particle size may be within the range from about 40 nm to about 5 micron, from about 50nm to about 1 micron, from about 75 nm to about 500 nm, or about 100 nm, for example.

The average molecular weight of the insoluble polysiloxane, the viscosity of the silicone emulsion, and the size of the particle comprising the insoluble polysiloxane are determined by methods commonly used by those skilled in the art, such as the methods disclosed in Smith, A. L. The Analytical Chemistry of Silicones, John Wiley & Sons, Inc.: New York, 1991. For example, the viscosity of the silicone emulsion can be measured at 30°C with a Brookfield viscometer with spindle 6 at 2.5 rpm. The silicone emulsion may further include an additional emulsifier together with the anionic surfactant,

Other classes of silicones suitable for use herein include but are not limited to: i) silicone fluids, including but not limited to, silicone oils, which are flowable materials having viscosity less than about 1,000,000 csk as measured at 25°C; ii) aminosilicones, which contain at least one primary, secondary or tertiary amine; iii) cationic silicones, which contain at least one quaternary ammonium functional group; iv) silicone gums; which include materials having viscosity greater or equal to 1,000,000 csk as measured at 25°C; v) silicone resins, which include highly crosslinked polymeric siloxane systems; vi) high refractive index silicones, having refractive index of at least 1.46, and vii) mixtures thereof.

The conditioning agent may also comprise at least one organic conditioning material such as oil or wax, either alone or in combination with other conditioning agents, such as the silicones described above. The organic material can be non-polymeric, oligomeric or polymeric. It may be in the form of oil or wax and may be added in the hair care composition neat or in a preemulsified form. Some non-limiting examples of organic conditioning materials include, but are not limited to: i) hydrocarbon oils; ii) polyolefins, iii) fatty esters, iv) fluorinated conditioning compounds, v) fatty alcohols, vi) alkyl glucosides and alkyl glucoside derivatives; vii) quaternary ammonium compounds; viii) polyethylene glycols and polypropylene glycols having a molecular weight of up to about 2,000,000 including those with CTFA names PEG-200, PEG-400, PEG-600, PEG-1000, PEG-2M, PEG-7M, PEG-14M, PEG-45M and mixtures thereof.

2. Emulsifiers

A variety of anionic and nonionic emulsifiers can be used in the hair care composition described herein. The anionic and nonionic emulsifiers can be either monomeric or polymeric in nature. Monomeric examples include, by way of illustrating and not limitation, alkyl ethoxylates, alkyl sulfates, soaps, and fatty esters and their derivatives. Polymeric examples include, by way of illustrating and not limitation, polyacrylates, polyethylene glycols, and block copolymers and their derivatives. Naturally occurring emulsifiers such as lanolins, lecithin and lignin and their derivatives are also non-limiting examples of useful emulsifiers.

3. Chelating Agents

The hair care composition described herein may also comprise a chelant. Suitable chelants include those listed in A E Martell & R M Smith, Critical Stability Constants, Vol. 1, Plenum Press, New York & London (1974) and A E Martell & R D Hancock, Metal Complexes in Aqueous Solution, Plenum Press, New York & London (1996) . When related to chelants, the term "salts and derivatives thereof" means the salts and derivatives comprising the same functional structure (e.g., same chemical backbone) as the chelant they are referring to and that have similar or better chelating properties. This term include alkali metal, alkaline earth, ammonium, substituted ammonium (i.e. monoethanolammonium, diethanolammonium, triethanolammonium) salts, esters of chelants having an acidic moiety and mixtures thereof, in particular all sodium, potassium or ammonium salts. The term "derivatives" also includes "chelating surfactant" compounds, such as those exemplified in U.S. Pat. No. 5,284,972, and large molecules comprising one or more chelating groups having the same functional structure as the parent chelants, such as polymeric EDDS (ethylenediaminedisuccinic acid) disclosed in U.S. Pat. No. 5,747,440.

Levels of the EDDS chelant in the hair care compositions may be as low as about 0.01 wt% or even as high as about 10 wt%. The level of the EDDS chelant may be at least about 0.05 wt%, at least about 0.1 wt%, at least about 0.25 wt%, at least about 0.5 wt%, at least about 1 wt%, or at least about 2 wt% by weight of the hair care composition. Levels above about 4 wt% can be used but may not result in additional benefit.

4. Anti-dandruff actives

Anti-dandruff agents suitable for use in hair care compositions include pyridinethione salts, azoles (e.g.,ketoconazole, econazole, and elubiol), octopirox, selenium sulfide, particulate sulfur, salicylic acid, and mixtures thereof. A typical anti-dandruff agent is pyridinethione salt. Hair care compositions can also include a zinc-containing layered material. An example of a zinc-containing layered material can include zinc carbonate materials. Of these, zinc carbonate and pyridinethione salts (particularly zinc pyridinethione or "ZPT) are common in the composition, and often present together.

5. Carrier

The hair care compositions can be in the form of a liquid (under ambient conditions). Such compositions comprise from 40% to 83% alternatively from about 45% to about 80% of a carrier, by weight of the hair care composition. The carrier may comprise water, or a miscible mixture of water and organic solvent. The carrier may comprise water with minimal or no significant concentrations of organic solvent, except as otherwise incidentally incorporated into the composition as minor ingredients of other essential or optional components.

The carrier may include water and water solutions of lower alkyl alcohols and polyhydric alcohols. The lower alkyl alcohols may be monohydric alcohols having 1 to 6 carbons, in one aspect, ethanol and isopropanol. Exemplary polyhydric may include propylene glycol, hexylene glycol, glycerin, and propane diol.

H. FOAM DISPENSER

The hair care composition described herein can be provided in a foam dispenser. The foam dispenser can be an aerosol foam dispenser. The aerosol foam dispenser can comprise a reservoir for holding the concentrated hair treatment composition. The reservoir may be made out of any suitable material selected from the group consisting of plastic, metal, alloy, laminate, and combinations thereof. In an embodiment, the reservoir may be for one-time use. In an embodiment, the reservoir may be removable from the aerosol foam dispenser. Alternatively, the reservoir may be integrated with the aerosol foam dispenser. In an embodiment, there may be two or more reservoirs.

In an embodiment, the reservoir may be comprised of a material selected from the group consisting of rigid materials, flexible materials, and combinations thereof. The reservoir is comprised of a rigid material if the reservoir material does not collapse under external atmospheric pressure when it is subject to an interior partial vacuum.

The foam dispenser may also be a mechanical foam dispenser. The mechanical foam dispenser described may be selected from the group consisting of squeeze foam dispensers, pump foam dispensers, other mechanical foam dispensers, and combinations thereof. In an embodiment, the mechanical foam dispenser is a squeeze foam dispenser. Non-limiting examples of suitable pump dispensers include those described in WO 2004/078903, WO 2004/078901, and WO 2005/078063 and may be supplied by Albea (60 Electric Ave., Thomaston, CT 06787 USA) or Rieke Packaging Systems (500 West Seventh St., Auburn, Indiana 46706).

The mechanical foam dispenser may comprise a reservoir for holding the concentrated hair treatment composition. The reservoir may be made out of any suitable material selected from the group consisting of plastic, metal, alloy, laminate, and combinations thereof. The reservoir may be a refillable reservoir such as a pour-in or screw-on reservoir, or the reservoir may be for one-time use. The reservoir may also be removable from the mechanical foam dispenser. Alternatively, the reservoir may be integrated with the mechanical foam dispenser. In an embodiment, there may be two or more reservoirs.

In an embodiment, the reservoir may be comprised of a material selected from the group consisting of rigid materials, flexible materials, and combinations thereof. The reservoir may be comprised of a rigid material if it does not collapse under external atmospheric pressure when it is subject to an interior partial vacuum.

I. PRODUCT FORM

The hair care composition descried herein may be presented in typical hair care formulations. They may be in the form of solutions, dispersions, emulsions, encapsulations, foams, and other delivery mechanisms. The hair care composition may be used as a hair tonic, a leave-on hair product, a styling product, a rinse-off product such as shampoos and personal cleansing products, a treatment product, and/or any other form that may be applied to hair.

DATA

Referring to Tables 1 and 2, the base formulations were made by mixing 1.6% perfume, 24% sodium undecyl sulfate (CAS# 1072-24-8) active, 6% lauramidopropyl betaine (CAS# 4292-10-8) active and 60.4% deionized water which leaves 8% unfilled for the addition of a viscosity reducing agent (the balance being filled in by distilled water). For all compositions, the surfactant, water and additives including viscosity reducing agents were expected to be in a single phase. The viscosity for formulations that showed hazing or clouding and compositions that appeared macroscopically heterogeneous (e.g. multiple layers) at room temperature were not included in the data (represented by N/A).

The agents from Tables 1 and 2 were added to the base formulation at a percentage of 2%, 4%, 6%, and 8% into the above surfactant solution. The subsequent formulations were vortexed and put into oven at 60 °C overnight to form homogeneous solution. The viscosities of the formulations were measured with calibrated viscometers (Size 200/350/450) from Cannon Instrument Company (2139 High Tech Road, State College, PA, USA, 16803). Prior to the measurement, the formulations were equilibrated in the viscometer reservoir for 30 min at 40 °C in water bath to ensure a homogeneous temperature was reached in the system.

After the equilibration, the formulations were drawn to reach the starting mark with a rubber suction bulb and the flow time between the starting mark and end mark was recorded for calculation. Each formulation was measured three times to calculate average and standard deviation. Between samples, viscometer was cleaned with water and acetone to rinse off residual. The results appear in Table 1.

Viscosity values were calculated based on the equation:

$$\text{Viscosity } (\text{mm}^2/\text{s. (cSt)}) = \text{Time (s)} * \text{Constant } (\text{mm}^2/\text{s}^2\text{. (cSt/s)})$$

The time in the above equation is the flow time recorded in the experiment and the constant numbers for each calibrated viscometer were obtained from the manuals.

The partition dispersion coefficient (PDC) was calculated using the following equation:

$$\text{PDC} = \log P - 0.3001 * (\delta D)^2 + 10.362 * \delta D - 93.251$$

wherein logP is the octanol water partitioning coefficient as computed by the Consensus algorithm implemented in ACD/Percepta version 14.02 by Advanced Chemistry Development, Inc. (ACD/Labs, Toronto, Canada), and wherein $\delta D$ is the Hansen solubility dispersion parameter in $(MPa)^{1/2}$ computed using Steven Abbott and Hiroshi Yamamoto's "HSPIP - Hansen Solubility Parameters in Practice" program, $4^{th}$ Edition, version 4.1.07.

Table 1

| Agent name | Lowest viscosity achieved (cSt) | PDC | Octanol/Water Partition Coefficient logP | # of polar group | Max # of acyclic interconnecting SP3 carbon atoms |
|---|---|---|---|---|---|
| Glycerin | 412 | -5.86 | -1.73 | 3 | 3 |
| Dipropylene Glycol | 53 | -4.49 | -0.62 | 3 | 3 |
| Ethanol | 29 | -4.37 | -0.23 | 1 | 2 |
| Raspberry Ketone | 33 | -2.92 | 1.43 | 2 | 2 |
| Triethyl Citrate | 41 | -2.75 | 1.09 | 4 | 2 |
| 5-Methyl-3-Heptanone Oxime | 246 | -2.54 | 2.00 | 1 | 5 |
| Hydroxycitronellal | 34 | -2.01 | 2.08 | 2 | 9 |
| Camphor Gum | 127 | -1.32 | 2.49 | 1 | 1 |
| 2-Isopropyl-5-methyl-2-hexenal | 332 | -1.25 | 3.20 | 1 | 4 |
| Eucalyptol | 203 | -1.08 | 2.85 | 1 | 1 |
| 1,1-Dimethoxyoctane | 308 | -0.96 | 3.58 | 2 | 7 |
| Isobutyl Hexanoate | 109 | -0.94 | 3.60 | 1 | 5 |
| Dihyro Iso Jasmonate | 119 | -0.92 | 2.89 | 2 | 6 |
| Cyclaprop | 404 | -0.50 | 3.31 | 2 | 2 |
| Orange Flower Ether | 419 | -0.30 | 3.78 | 2 | 3 |
| Iso Butyl Salicylate | 409 | -0.30 | 3.67 | 2 | 4 |
| Butyl Salicylate | 368 | -0.21 | 3.76 | 2 | 4 |
| Citronellyl Acetate | 363 | -0.02 | 4.07 | 1 | 6 |
| 3,7-Dimethyloctyl Acetate | 446 | 0.01 | 4.55 | 1 | 10 |
| Iso Bornyl Propionate | 463 | 0.01 | 3.95 | 1 | 2 |
| Veloutone | 47 | 0.10 | 4.00 | 1 | 5 |
| Isoamyl Salicylate | 193 | 0.13 | 4.02 | 2 | 4 |
| gamma-Terpinene | 32 | 0.20 | 4.10 | 2 | 3 |
| Linalyl Iso Butyrate | 38 | 0.27 | 4.41 | 2 | 4 |
| alpha - Terpinene | 42 | 0.44 | 4.38 | 2 | 3 |
| Limonene | 86 | 0.50 | 4.40 | 2 | 1 |
| Dipentene | 35 | 0.50 | 4.40 | 2 | 1 |
| Geranyl Phenyl Acetate | 34 | 1.65 | 5.47 | 1 | 2 |

(continued)

| Agent name | Lowest viscosity achieved (cSt) | PDC | Octanol/Water Partition Coefficient logP | # of polar group | Max # of acyclic interconnecting SP3 carbon atoms |
|---|---|---|---|---|---|
| Iso Propyl Myristate | 30 | 3.13 | 7.41 | 1 | 13 |
| Hexadecane | 18 | 4.37 | 8.74 | 0 | 16 |
| 1-Eicosene | N/A | 5.58 | 9.94 | 1 | 18 |

Table 2

| Kinematic viscosity at 40 C [cSt] | | | | | | | |
|---|---|---|---|---|---|---|---|
| Name | 2% oil | 4% oil | 6% oil | 8 % oil | Lowest viscosity achieved | PDC | Octanol/Water Partition Coefficient logP |
| Glycerin | 649.2 | 579.7 | 512.3 | 411.5 | 412 | -5.86 | -1.73 |
| Dipropylene Glycol | 321.6 | 141.8 | 109.8 | 52.9 | 53 | -4.49 | -0.62 |
| Ethanol | 205.6 | 111.0 | 50.8 | 29.2 | 29 | -4.37 | -0.23 |
| Raspberry Ketone | 151.9 | 107.4 | 54.0 | 33.0 | 33 | -2.92 | 1.43 |
| Triethyl Citrate | 269.4 | 136.6 | 66.6 | 40.5 | 41 | -2.75 | 1.09 |
| 5-Methyl-3-Heptanone Oxime | 246.3 | N/A | N/A | N/A | 246 | -2.54 | 2.00 |
| Hydroxycitronellal | 310.8 | 145.4 | 65.9 | 34.1 | 34 | -2.01 | 2.08 |
| Camphor Gum | 365.8 | 127.2 | N/A | N/A | 127 | -1.32 | 2.49 |
| 2-Isopropyl-5-methyl-2-hexenal | 332.3 | N/A | N/A | N/A | 332 | -1.25 | 3.20 |
| Eucalyptol | 527.3 | 292.9 | 203.1 | N/A | 203 | -1.08 | 2.85 |
| 1,1-Dimethoxyoctane | 500.7 | 308.1 | N/A | N/A | 308 | -0.96 | 3.58 |
| Isobutyl Hexanoate | 390.0 | 177.9 | 140.9 | 109.4 | 109 | -0.94 | 3.60 |
| Dihyro Iso Jasmonate | 334.9 | 119.1 | N/A | N/A | 119 | -0.92 | 2.89 |
| Cyclaprop | 404.2 | N/A | N/A | N/A | 404 | -0.50 | 3.31 |
| Orange Flower Ether | 552.2 | 418.8 | N/A | N/A | 419 | -0.30 | 3.78 |
| Iso Butyl Salicylate | 408.7 | N/A | N/A | N/A | 409 | -0.30 | 3.67 |
| Butyl Salicylate | 367.9 | N/A | N/A | N/A | 368 | -0.21 | 3.76 |
| Citronellyl Acetate | 362.7 | N/A | N/A | N/A | 363 | -0.02 | 4.07 |
| 3,7-Dimethyloctyl Acetate | 445.7 | N/A | N/A | N/A | 446 | 0.01 | 4.55 |
| Iso Bornyl Propionate | 463.1 | N/A | N/A | N/A | 463 | 0.01 | 3.95 |
| Veloutone | 260.2 | 129.1 | 56.3 | 47.3 | 47 | 0.10 | 4.00 |
| Isoamyl Salicylate | 460.3 | 193.2 | N/A | N/A | 193 | 0.13 | 4.02 |
| gamma- Terpinene | 238.0 | 65.0 | 31.7 | N/A | 32 | 0.20 | 4.10 |

(continued)

| Kinematic viscosity at 40 C [cSt] | | | | | | | |
|---|---|---|---|---|---|---|---|
| Name | 2% oil | 4% oil | 6% oil | 8 % oil | Lowest viscosity achieved | PDC | Octanol/Water Partition Coefficient logP |
| Linalyl Iso Butyrate | 223.4 | 73.0 | 37.7 | 39.9 | 38 | 0.27 | 4.41 |
| alpha - Terpinene | 324.4 | 96.0 | 41.5 | 46.7 | 42 | 0.44 | 4.38 |
| Limonene | 366.5 | 166.8 | 97.5 | 86.4 | 86 | 0.50 | 4.40 |
| Dipentene | 307.6 | 74.5 | 35.1 | 42.6 | 35 | 0.50 | 4.40 |
| Geranyl Phenyl Acetate | 224.0 | 76.0 | 34.3 | 36.6 | 34 | 1.65 | 5.47 |
| Iso Propyl Myristate | 234.2 | 53.7 | 30.4 | 45.0 | 30 | 3.13 | 7.41 |
| Hexadecane | 21.1 | 18.1 | N/A | N/A | 18 | 4.37 | 8.74 |
| 1-Eicosene | N/A | N/A | N/A | N/A | N/A | 5.58 | 9.94 |

Now referring to Tables 3 and 4, the following surfactant formulations were made by mixing 1.6% perfume, 24% sodium undecyl sulfate (CAS# 1072-24-8) active, 6% lauramidopropyl betaine (CAS# 4292-10-8) active, and 60.4% deionized water which leaves 8% unfilled for the addition of the listed agents (the balance being filled in by distilled water). The agents were added at a percentage of 2%, 4%, 6%, and 8% into the above surfactant formulation. The resulting formulations were vortexed and put into oven at 60 °C overnight to form homogeneous solution. For all formulations, the surfactant, water and additives, including the added agents, were expected to be in a single phase. Samples that showed hazing or clouding and formulas that appeared macroscopically heterogeneous (e.g. multiple layers) at room temperature were not considered for further analysis and were labeled "N/A."

The resulting formulations were weighed out in a scintillation vial combined with 10 ml of water to achieve a representative dilution during use: 0.5g formula, 10 ml deionized water for 20:1 dilution. After the combination of the ingredients, the vial was vigorously shaken by hand and the samples were visually inspected by looking at the solution against a dark background. Samples that appeared cloudy (turbid) within less than a day were noted as cloudy ("Yes"). Samples that did not appear cloudy (turbid) within less than a day were noted as not cloudy ("No"). Samples that appeared cloudy at the 20:1 dilution for any of the 2%, 4% or 6% concentration of oil may be most desirable by requiring a limited amount of the agent to be added, limiting cost and trade-offs associated with higher levels.

Table 3 shows that the agents with a PDC value of from about 0.05 to about 2.0, alternatively from about 0.08 to about 1.9, alternatively from about 0.09 to about 1.8, alternatively from about 0.095 to about 1.7 appear cloudy at dilution at a concentration of 2%, 4%, and/or 6%. The appearance of a cloudy formulation at 1:20 dilution at 2%, 4%, and/or 6% can signal improved deposition of surfactant soluble active agents during consumer use.

Table 3

| Name | Cloudy at 1:20 dilution at 2%, 4%, and/or 6% | Octanol/Water Partition Coefficient logP | PDC |
|---|---|---|---|
| 2-Isopropyl-5-methyl-2- | No | 3.20 | -1.25 |
| hexenal | | | |
| Eucalyptol | No | 2.85 | -1.08 |
| Isobutyl Hexanoate | No | 3.60 | -0.94 |
| Dihyro Iso Jasmonate | No | 2.89 | -0.92 |
| Cyclaprop | No | 3.31 | -0.50 |
| Iso Butyl Salicylate | No | 3.67 | -0.30 |
| Butyl Salicylate | No | 3.76 | -0.21 |
| Citronellyl Acetate | No | 4.07 | -0.02 |

(continued)

| Name | Cloudy at 1:20 dilution at 2%, 4%, and/or 6% | Octanol/Water Partition Coefficient logP | PDC |
|---|---|---|---|
| 3,7-Dimethyloctyl Acetate | No | 4.55 | 0.01 |
| Veloutone | Yes | 4.00 | 0.10 |
| Isoamyl Salicylate | Yes | 4.02 | 0.13 |
| gamma- Terpinene | Yes | 4.10 | 0.20 |
| Linalyl Iso Butyrate | Yes | 4.41 | 0.27 |
| alpha-Terpinene | Yes | 4.38 | 0.44 |
| Limonene | Yes | 4.40 | 0.50 |
| Dipentene | Yes | 4.40 | 0.50 |
| Geranyl Phenyl Acetate | Yes | 5.47 | 1.65 |
| Iso Propyl Myristate | Yes | 7.41 | 3.13 |
| Hexadecane | No | 8.74 | 4.37 |

Table 4

| Name | Added at 2%: cloudy at 20:1 dilution | Added at 4%: cloudy at 20:1 dilution | Added at 6%: cloudy at 20:1 dilution | Added at 8%: cloudy at 20:1 dilution |
|---|---|---|---|---|
| 2-Isopropyl-5-methyl-2-hexenal | No | No | N/A | N/A |
| Eucalyptol | No | No | No | N/A |
| Isobutyl Hexanoate | No | No | No | Yes |
| Dihyro Iso Jasmonate | No | N/A | N/A | N/A |
| Cyclaprop | No | No | N/A | N/A |
| Iso Butyl Salicylate | No | No | N/A | N/A |
| Butyl Salicylate | No | N/A | N/A | N/A |
| Citronellyl Acetate | No | N/A | N/A | N/A |
| 3,7-Dimethyloctyl Acetate | No | N/A | N/A | N/A |
| Veloutone | No | No | Yes | Yes |
| Isoamyl Salicylate | No | Yes | N/A | N/A |
| gamma-Terpinene | No | No | Yes | Yes |
| Linalyl Iso Butyrate | No | No | Yes | Yes |
| alpha - Terpinene | No | No | Yes | Yes |
| Limonene | No | No | Yes | Yes |
| Dipentene | No | No | Yes | Yes |
| Geranyl Phenyl Acetate | Yes | Yes | Yes | Yes |
| Iso Propyl Myristate | No | No | Yes | Yes |
| Hexadecane | No | No | N/A | N/A |

TEST METHODS

A. Viscosity Method

The hair care composition described herein can have a viscosity of from about 10 cSt to about 2,000 cSt, alternatively from about 10 cSt to about 1,000 cSt, alternatively from about 10 cSt to about 500 cSt, alternatively from about 15 cSt to about 400 cSt, alternatively from about 20 cSt to about 300 cSt, alternatively from about 25 cSt to about 250 cSt, and alternatively from about 30 cSt to about 250 cSt.

The viscosity of the hair care composition can be calculated using the following method: Combine ingredients including surfactants, perfumes, viscosity reducing agents, polymers, other ingredients and the aqueous medium in a vessel. Samples are vortexed and placed into oven at 60 °C overnight to form a homogeneous solution. Samples that show hazing or clouding and formulas that appear macroscopically heterogeneous (e.g. multiple layers) at room temperature are not considered for further analysis and evaluation.

The viscosities of the formulations are measured with calibrated viscometers (Size 200/350/450) from Cannon Instrument Company (2139 High Tech Road, State College, PA, USA, 16803). Prior to the measurement, the formulations are equilibrated in the viscometer reservoir for 30 min at 40 °C in water bath to ensure a homogeneous temperature is reached in the system.

After the equilibration, the formulations are drawn to reach the starting mark with a rubber suction bulb and the flow time between the starting mark and end mark is recorded for calculation. Each formulation is measured three times to calculate average and standard deviation. Between samples, the viscometer is cleaned with water and acetone to rinse off residual.

Viscosities can be calculated based on the equation:

$$\text{Viscosity } (\text{mm}^2/\text{s}. (\text{cSt})) = \text{Time (s)} * \text{Constant } (\text{mm}^2/\text{s}^2. (\text{cSt/s}))$$

The time in the above equation is the flow time recorded in the experiment and the constants for each calibrated viscometer are obtained from the manuals.

EXAMPLES

The following examples illustrate embodiments of the hair care composition described herein. The exemplified hair care compositions can be made by mixing together water and surfactants along with any solids that need to be melted at an elevated temperature, e.g. about ambient temperature. Additional ingredients, including electrolytes, polymers, silicone emulsions, preservatives and fragrances may be added to the cooled product. It will be appreciated that other modifications of the hair care compositions within the skill of those in the formulation art can be undertaken. All parts, percentages, and ratios herein are by weight unless otherwise specified. Some components may come from suppliers as dilute solutions. The amount stated reflects the weight percent of the active material, unless otherwise specified.

Example BM6 is according to the invention. The other examples are illustrating examples and not part of the present invention.

Table 5

| Ingredient | Ex. BH4 | Ex. BI4 | Ex. BJ4 | Ex.BK4 | BN4 |
|---|---|---|---|---|---|
| Sodium Undecyl Sulfate[1] | 24 | 24 | 24 | 24 | 24 |
| Lauramidopropyl Betaine[2] | 6 | 6 | 6 | 6 | 6 |
| Isoamyl Salicylate | | 4 | | | |
| gamma-Terpinene | | | 6 | | |
| Linalyl Iso Butyrate | | | | 6 | |
| Geranyl phenyl acetate | 2 | | | | 4 |
| Fragrance | 2.4 | 2.4 | 2.4 | 2.4 | 2.4 |
| Citric acid | Adjust as needed for pH | | | | |

(continued)

| Ingredient | Ex. BH4 | Ex. BI4 | Ex. BJ4 | Ex.BK4 | BN4 |
|---|---|---|---|---|---|
| Water | Q.S. | Q.S. | Q.S. | Q.S. | Q.S. |
| 1. Sodium Undecyl Sulfate (C11, Neodol-1) at 70% active: supplied by P&G<br>2. LAPB (Mackam DAB), at 35% active level; supplied by Rhodia | | | | | |

Table 6

| Ingredient | Ex. BH6 | Ex. BI6 | Ex. BL6 | Ex. BM6 | BN6 |
|---|---|---|---|---|---|
| Sodium Undecyl Sulfate[1] | 24 | 24 | 24 | 24 | 24 |
| Lauramidopropyl Betaine[2] | 6 | 6 | 6 | 6 | 6 |
| Veloutone | 6 | | | | |
| alpha - Terpinene | | | 6 | | |
| Limonene | | | | 6 | |
| Dipentene | | | | | 6 |
| Geranyl phenyl acetate | | 6 | | | |
| Fragrance | 2.4 | 2.4 | 2.4 | 2.4 | 2.4 |
| Citric acid | Adjust as needed for pH | | | | |
| Water | Q.S. | Q.S. | Q.S. | Q.S. | Q.S. |
| 1. Sodium Undecyl Sulfate (C11, Neodol-1) at 70% active: supplied by P&G<br>2. LAPB (Mackam DAB), at 35% active level; supplied by Rhodia | | | | | |

Table 7

| Ingredient | Ex. BH8 | Ex. BJ8 | Ex.BK8 | Ex. BL8 | Ex. BM8 | BN8 |
|---|---|---|---|---|---|---|
| Sodium Undecyl Sulfate[1] | 24 | 24 | 24 | 24 | 24 | 24 |
| Lauramidopropyl Betaine[2] | 6 | 6 | 6 | 6 | 6 | 6 |
| Veloutone | 8 | | | | | |
| gamma- Terpinene | | 8 | | | | |
| Linalyl Iso Butyrate | | | 8 | | | |
| alpha - Terpinene | | | | 8 | | |
| Limonene | | | | | 8 | |
| Geranyl Phenyl Acetate | | | | | | 8 |
| Fragrance | 2.4 | 2.4 | 2.4 | 2.4 | 2.4 | 2.4 |
| Citric acid | Adjust as needed for pH | | | | | |
| Water | Q.S. | Q.S. | Q.S. | Q.S. | Q.S. | Q.S. |
| 1. Sodium Undecyl Sulfate (C11, Neodol-1) at 70% active: supplied by P&G<br>2. LAPB (Mackam DAB), at 35% active level; supplied by Rhodia | | | | | | |

## Claims

**1.** A hair care composition comprising:

a) from 0.2% to 10% of one or more surfactant soluble active agents, by weight of the hair care composition, wherein the surfactant soluble active agent is a perfume oil, wherein the total amount of one or more surfactant soluble active agents is from 0.2% to 10%, by weight of the hair care composition;
b) from 4% to 7% of one or more viscosity reducing agents, by weight of the hair care composition, having a partition dispersion coefficient of from 0.05 to 2.0, wherein the one or more viscosity reducing agents comprises limonene, wherein the total amount of one or more viscosity reducing agents is from 4% to 7%, by weight of the hair care composition,
wherein the "partition dispersion coefficient" is defined by the following equation:

$$PDC = \log P - 0.3001 * (\delta D)^2 + 10.362 * \delta D - 93.251,$$

wherein logP is the octanol water partitioning coefficient as computed by the Consensus algorithm implemented in ACD/Percepta version 14.02 by Advanced Chemistry Development, Inc. (ACD/Labs, Toronto, Canada), and wherein $\delta D$ is the Hansen solubility dispersion parameter in $(MPa)^{1/2}$ computed using Steven Abbott and Hiroshi Yamamoto's "HSPIP - Hansen Solubility Parameters in Practice" program, 4th Edition, version 4.1.07;
c) from 20% to 32% of one or more anionic surfactants, by weight of the hair care composition;
d) from 40% to 83% of a carrier, by weight of the hair care composition;

wherein the one or more viscosity reducing agents has a molecular weight of from 100 g/mol (daltons) to 300 g/mol (daltons); and
wherein the hair care composition has a liquid phase kinematic viscosity, measured at 40 degrees Celsius, of from 10 $mm^2/s$ (cSt) to 1,000 $mm^2/s$ (cSt).

**2.** The hair care composition according to claim 1, wherein the hair care composition further comprises from 0.25% to 14% of one or more co-surfactants, by weight of the hair care composition, selected from the group consisting of amphoteric, non-ionic, zwitterionic, and combinations thereof.

**3.** The hair care composition according to any preceding claim, wherein the partition dispersion coefficient of the one or more viscosity reducing agents is from 0.08 to 1.9.

**4.** The hair care composition according to any preceding claim, wherein the hair care composition comprises less than 4% of a counteracting additive having a partition dispersion coefficient of from -3.1 to -0.7, by weight of the hair care composition; and wherein the weight ratio of the one or more viscosity reducing agents to the counteracting additive is greater than 2:1.

**5.** The hair care composition according to any preceding claim, wherein the weight ratio of the one or more viscosity reducing agents to the counteracting additive is greater than 5:1.

**6.** The hair care composition according to any preceding claim, wherein the hair care composition comprises less than 0.5% of a counteracting additive having a partition dispersion coefficient of from -3.1 to -0.7, by weight of the hair care composition.

**7.** The hair care composition according to any preceding claim, wherein the liquid phase kinematic viscosity is from 15 $mm^2/s$ (cSt) to 400 $mm^2/s$ (cSt).

**8.** The hair care composition according to any preceding claim, wherein the hair care composition comprises from 3.5% to 8% of the one or more viscosity reducing agents, by weight of the hair care composition.

**9.** The hair care composition according to any preceding claim, wherein the hair care composition further comprises a cationic polymer selected from the group consisting of guar polymers, non-guar galactomannan polymers, tapioca polymers, copolymers of acrylamide monomers and cationic monomers, cellulose polymers, and combinations thereof.

**10.** A method of treating the hair, the method comprising:

a) providing the hair care composition according to any preceding claim in a foam dispenser;
b) dispensing the hair care composition from the foam dispenser as a foam;

c) applying the foam to the hair; and
d) rinsing the foam from the hair;

wherein the foam has a density of from 0.05 g/$cm^3$ to 0.30 g/$cm^3$ when dispensed from the foam dispenser.

**Patentansprüche**

1. Haarpflegezusammensetzung, umfassend:

a) zu 0,2 Gew.-% bis 10 Gew.-% ein oder mehrere tensidlösliche Wirkstoffe, bezogen auf das Gewicht der Haarpflegezusammensetzung, wobei der tensidlösliche Wirkstoff ein Parfümöl ist, wobei die Gesamtmenge eines oder mehrerer tensidlöslicher Wirkstoffe von 0,2 Gew.-% bis 10 Gew.-% der Haarpflegezusammensetzung beträgt;
b) zu 4 Gew.-% bis 7 Gew.-% ein oder mehrere Viskositätsreduktionsmittel, bezogen auf das Gewicht der Haarpflegezusammensetzung, die einen Aufteilungsdispersionskoeffizienten von 0,05 bis 2,0 aufweisen,

wobei das eine oder die mehreren Viskositätsreduktionsmittel Limonen umfassen,
wobei die Gesamtmenge eines oder mehrerer Viskositätsreduktionsmittel von 4 Gew.-% bis 7 Gew.-%, bezogen auf das Gewicht der Haarpflegezusammensetzung, beträgt,
wobei der "Aufteilungsdispersionskoeffizient" durch die folgende Gleichung definiert ist:

$$PDC= \log P - 0{,}3001 * (\delta D)^2 + 10{,}362 * \delta D - 93{,}251,$$

wobei logP der Octanol-Wasser-Aufteilungskoeffizient ist, berechnet mittels des von Advanced Chemistry Development, Inc. (ACD/Labs, Toronto, Kanada) in der ACD/Percepta-Version 14.02 implementierten Konsensus-Algorithmus, und wobei δD der Hansen-Löslichkeitsdispersionsparameter in $(MPa)^{1/2}$ ist, berechnet unter Verwendung von Steven Abbotts und Hiroshi Yamamotos Programm "HSPIP - Hansen Solubility Parameter in Practice", 4. Auflage, Version 4.1.07;

c) zu 20 Gew.-% bis 32 Gew.-% ein oder mehrere anionische Tenside, bezogen auf das Gewicht der Haarpflegezusammensetzung;
d) zu 40 Gew.-% bis 83 Gew.-% einen Träger, bezogen auf das Gewicht der Haarpflegezusammensetzung;

wobei das eine oder die mehreren Viskositätsreduktionsmittel ein Molekulargewicht von 100 g/mol (Dalton) bis 300 g/mol (Dalton) aufweist; und
wobei die Haarpflegezusammensetzung eine kinematische Viskosität der Flüssigphase, gemessen bei 40 Grad Celsius, von 10 $mm^2$/s (cSt) bis 1.000 $mm^2$/s (cSt) aufweist.

2. Haarpflegezusammensetzung nach Anspruch 1, wobei die Haarpflegezusammensetzung ferner zu 0,25 Gew.-% bis 14 Gew.-% ein oder mehrere Co-Tenside, bezogen auf das Gewicht der Haarpflegezusammensetzung, umfasst, ausgewählt aus der Gruppe bestehend aus amphoteren, nichtionischen, zwitterionischen Stoffen und Kombinationen davon.

3. Haarpflegezusammensetzung nach einem der vorstehenden Ansprüche, wobei der Aufteilungsdispersionskoeffizient des einen oder der mehreren Viskositätsreduktionsmittel von 0,08 bis 1,9 beträgt.

4. Haarpflegezusammensetzung nach einem der vorstehenden Ansprüche, wobei die Haarpflegezusammensetzung zu weniger als 4 Gew.-% einen entgegenwirkenden Zusatzstoff, der einen Aufteilungsdispersionskoeffizienten von -3,1 bis -0,7 aufweist, umfasst, bezogen auf das Gewicht der Haarpflegezusammensetzung; und wobei das Gewichtsverhältnis des einen oder der mehreren Viskositätsreduktionsmittel zu dem entgegenwirkenden Zusatzstoff größer als 2 : 1 ist.

5. Haarpflegezusammensetzung nach einem der vorstehenden Ansprüche, wobei das Gewichtsverhältnis des einen oder der mehreren Viskositätsreduktionsmittel zu dem entgegenwirkenden Zusatzstoff größer als 5 : 1 ist.

6. Haarpflegezusammensetzung nach einem der vorstehenden Ansprüche, wobei die Haarpflegezusammensetzung

zu weniger als 0,5 Gew.-% einen entgegenwirkenden Zusatzstoff, der einen Aufteilungsdispersionskoeffizienten von -3,1 bis -0,7 aufweist, umfasst, bezogen auf das Gewicht der Haarpflegezusammensetzung.

**7.** Haarpflegezusammensetzung nach einem der vorstehenden Ansprüche, wobei die kinematische Viskosität der Flüssigphase von 15 $mm^2/s$ (cSt) bis 400 $mm^2/s$ (cSt) beträgt.

**8.** Haarpflegezusammensetzung nach einem der vorstehenden Ansprüche, wobei die Haarpflegezusammensetzung zu 3,5 Gew.-% bis 8 Gew.-% das eine oder die mehreren Viskositätsreduktionsmittel umfasst, bezogen auf das Gewicht der Haarpflegezusammensetzung.

**9.** Haarpflegezusammensetzung nach einem der vorstehenden Ansprüche, wobei die Haarpflegezusammensetzung ferner ein kationisches Polymer, ausgewählt aus der Gruppe bestehend aus Guargummi-Polymeren, Nicht-Guargummi-Galactomannan-Polymeren, Tapioka-Polymeren, Copolymeren von Acrylamid-Monomeren und kationischen Monomeren, Cellulose-Polymeren und Kombinationen davon, umfasst.

**10.** Verfahren zum Behandeln des Haars, das Verfahren umfassend:

a) Bereitstellen der Haarpflegezusammensetzung nach einem der vorstehenden Ansprüche in einem Schaumspender;
b) Abgeben der Haarpflegezusammensetzung aus dem Schaumspender als ein Schaum;
c) Aufbringen des Schaums auf das Haar; und
d) Spülen des Schaums aus dem Haar;
wobei der Schaum beim Abgeben aus dem Schaumspender eine Dichte von 0,05 $g/cm^3$ bis 0,30 $g/cm^3$ aufweist.

## Revendications

**1.** Composition pour soins capillaires, comprenant :

a) de 0,2 % à 10 % d'un ou plusieurs agents actifs solubles dans un tensioactif, en poids de la composition pour soins capillaires, dans laquelle l'agent actif soluble dans un tensioactif est une huile parfumée, dans laquelle la quantité totale d'un ou plusieurs agents actifs solubles dans un tensioactif va de 0,2 % à 10 %, en poids de la composition pour soins capillaires ;
b) de 4 % à 7 % d'un ou plusieurs agents réducteurs de viscosité, en poids de la composition pour soins capillaires, ayant un coefficient de dispersion de partage allant de 0,05 à 2,0,

dans laquelle le ou les agents réducteurs de viscosité comprennent du limonène,
dans laquelle la quantité totale d'un ou plusieurs agents réducteurs de viscosité va de 4 % à 7 %, en poids de la composition pour soins capillaires,
dans laquelle le « coefficient de dispersion de partage » est défini par l'équation suivante :

$$PDC= \log P - 0,3001 * (\delta D)^2 + 10,362 * \delta D - 93,251,$$

dans laquelle logP est le coefficient de partage octanol-eau tel que calculé par l'algorithme Consensus implémenté dans ACD/Percepta version 14.02 par Advanced Chemistry Development, Inc. (ACD/Labs, Toronto, Canada), et dans laquelle $\delta D$ est le paramètre de dispersion de solubilité de Hansen en $(MPa)^{1/2}$ calculé en utilisant le programme « HSPIP - Hansen Solubility Parameters in Practice - Paramètres de solubilité Hansen dans la pratique », 4ème Édition, version 4.1.07 de Steven Abbott et Hiroshi Yamamoto ;

c) de 20 % à 32 % d'un ou plusieurs agents tensioactifs anioniques, en poids de la composition pour soins capillaires ;
d) de 40 % à 83 % d'un véhicule, en poids de la composition pour soins capillaires ;

dans laquelle le ou les agents réducteurs de viscosité ont une masse moléculaire allant de 100 g/mol (daltons) à 300 g/mol (daltons) ; et
dans laquelle la composition pour soins capillaires a une viscosité cinématique de phase liquide, mesurée à 40 degrés Celsius, allant de 10 $mm^2/s$ (cSt) à 1000 $mm^2/s$ (cSt).

**2.** Composition pour soins capillaires selon la revendication 1, dans laquelle la composition pour soins capillaires comprend en outre de 0,25 % à 14 % d'un ou plusieurs cotensioactifs, en poids de la composition pour soins capillaires, choisis dans le groupe constitué d'amphotères, non ioniques, zwittérioniques, et combinaisons de ceux-ci.

**3.** Composition pour soins capillaires selon une quelconque revendication précédente, dans laquelle le coefficient de dispersion de partage du ou des agents réducteurs de viscosité va de 0,08 à 1,9.

**4.** Composition pour soins capillaires selon une quelconque revendication précédente, dans laquelle la composition pour soins capillaires comprend moins de 4 % d'un additif antagoniste ayant un coefficient de dispersion de partage allant de -3,1 à -0,7, en poids de la composition pour soins capillaires ; et dans laquelle le rapport pondéral du ou des agents réducteurs de viscosité à l'additif antagoniste est supérieur à 2:1.

**5.** Composition pour soins capillaires selon une quelconque revendication précédente, dans laquelle le rapport pondéral du ou des agents réducteurs de viscosité à l'additif antagoniste est supérieur à 5:1.

**6.** Composition pour soins capillaires selon une quelconque revendication précédente, dans laquelle la composition pour soins capillaires comprend moins de 0.5 % d'un additif antagoniste ayant un coefficient de dispersion de partage allant de -3,1 à -0,7, en poids de la composition pour soins capillaires.

**7.** Composition pour soins capillaires selon une quelconque revendication précédente, dans laquelle la viscosité cinématique de phase liquide va de 15 $mm^2/s$ (cSt) à 400 $mm^2/s$ (cSt).

**8.** Composition pour soins capillaires selon une quelconque revendication précédente, dans laquelle la composition pour soins capillaires comprend de 3,5 % à 8 % du ou des agents réducteurs de viscosité, en poids de la composition pour soins capillaires.

**9.** Composition pour soins capillaires selon une quelconque revendication précédente, dans laquelle la composition pour soins capillaires comprend en outre un polymère cationique choisi dans le groupe constitué de polymères de gomme de guar, polymères de galactomannanes autres que gomme de guar, polymères de tapioca, copolymères de monomères acrylamide et de monomères cationiques, polymères de cellulose, et combinaisons de ceux-ci.

**10.** Procédé de traitement des cheveux, le procédé comprenant :

a) la fourniture de la composition pour soins capillaires selon une quelconque revendication précédente dans un distributeur de mousse ;
b) la distribution de la composition pour soins capillaires à partir du distributeur de mousse en tant que mousse ;
c) l'application de la mousse sur les cheveux ; et
d) le rinçage de la mousse des cheveux ;
dans lequel la mousse a une masse volumique allant de 0,05 $g/cm^3$ à 0,30 $g/cm^3$ lorsqu'elle est distribuée à partir du distributeur de mousse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150071977 A1
- US 5607980 A, McAtee
- US 6217888 B, Oblong
- US 5686082 A
- US 5686367 A
- US 2486921 A
- US 2486922 A
- US 2396278 A
- US 3929678 A
- US 2658072 A
- US 2438091 A
- US 2528378 A
- US 34584 A
- US 5104646 A
- US 5106609 A
- US 4476282 A
- US 20070276087
- US 5284972 A
- US 5747440 A
- WO 2004078903 A
- WO 2004078901 A
- WO 2005078063 A


### Non-patent literature cited in the description


- McCutcheion's Detergents and Emulsifiers. Allured Publishing Corp, 1986
- McCutcheion's Functional Materials. 1992
- McCutcheon's, Emulsifiers and Detergents. M. C. Publishing Co, 1989
- **SOLAREK, D. B.** Cationic Starches in Modified Starches: Properties and Uses. CRC Press, Inc, 1986, 113-125
- **QIN-JI PENG ; ARTHUR S. PERLIN.** Observation on NMR Spectra of Starches in Dimethyl Sulfoxide, Iodine-Complexing, and Solvating in Water-Dimethyl Sulfoxide. *Carbohydrate Research,* 1987, vol. 160, 57-72
- **J. HOWARD BRADBURY ; J. GRANT COLLINS.** An Approach to the Structural Analysis of Oligosaccharides by NMR Spectroscopy. *Carbohydrate Research,* 1979, vol. 71, 15-25
- CTFA Cosmetic Ingredient Handbook. Cosmetic, Toiletry, and Fragrance Association, Inc, 2004
- Encyclopedia of Polymer Science and Engineering. John Wiley & Sons, Inc, 1989, vol. 15, 204-308
- **SMITH, A. L.** The Analytical Chemistry of Silicones. John Wiley & Sons, Inc, 1991
- **A E MARTELL ; R M SMITH.** Critical Stability Constants. Plenum Press, 1974, vol. 1
- **A E MARTELL ; R D HANCOCK.** Metal Complexes in Aqueous Solution. Plenum Press, 1996